(19) Europäisches Patentamt / European Patent Office / Office européen des brevets

(11) **EP 2 804 634 B1**

(12) **EUROPEAN PATENT SPECIFICATION**

(45) Date of publication and mention of the grant of the patent:
**28.08.2019 Bulletin 2019/35**

(51) Int Cl.:
*A61K 47/30* (2006.01)      *A61K 9/08* (2006.01)
*A61K 9/16* (2006.01)      *A61K 39/395* (2006.01)
*G01N 33/544* (2006.01)

(21) Application number: **13739077.9**

(22) Date of filing: **18.01.2013**

(86) International application number:
**PCT/US2013/022057**

(87) International publication number:
**WO 2013/109825 (25.07.2013 Gazette 2013/30)**

(54) **CHROMATOGRAPHIC MEDIA FOR STORAGE AND DELIVERY OF THERAPEUTIC BIOLOGICS AND SMALL MOLECULES**

CHROMATOGRAFISCHE MEDIEN ZUR LAGERUNG UND ABGABE VON THERAPEUTISCHEN BIOLOGISCHEN STOFFEN UND KLEINEN MOLEKÜLEN

MATÉRIAUX CHROMATOGRAPHIQUES POUR LE STOCKAGE ET LA DÉLIVRANCE D'AGENTS BIOLOGIQUES THÉRAPEUTIQUES ET DE PETITES MOLÉCULES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.01.2012 US 201261588312 P**

(43) Date of publication of application:
**26.11.2014 Bulletin 2014/48**

(73) Proprietor: **Natrix Separations Inc.**
**Burlington, ON L7L 6A8 (CA)**

(72) Inventors:
- **CHICKOSKY, John, A.**
  **Acton, MA 01720 (US)**
- **HONEYMAN, Charles, H.**
  **Toronto, ON M8V 2N8 (CA)**
- **MCGLAUGHLIN, Molly, S.**
  **Marion, MA 02738 (US)**
- **RAGHEB, Amro**
  **Mississauga, ON L5A 4A5 (CA)**

(74) Representative: **HGF Limited**
**1 City Walk**
**Leeds LS11 9DX (GB)**

(56) References cited:
WO-A1-95/33553          US-A1- 2003 166 594
US-A1- 2004 028 733     US-A1- 2009 029 438
US-A1- 2010 178 210

- KANANI ET AL: "Separation of human plasma proteins HSA and HIgG using high-capacity macroporous gel-filled membranes", BIOCHEMICAL ENGINEERING JOURNAL, ELSEVIER, AMSTERDAM, NL, vol. 35, no. 3, 31 May 2007 (2007-05-31), pages 295-300, XP022100393, ISSN: 1369-703X, DOI: 10.1016/J.BEJ.2007.01.034

## Description

## BACKGROUND OF THE INVENTION

Many bio-molecules, small molecules, drugs, and other therapeutic agents are unstable and require expensive isolation procedures, such as lyophilisation, to achieve adequate shelf life. Formulating pre-measured dosage forms of these agents is also a challenge. Moreover, even once isolated correctly, delivery of the therapeutic agent to a patient still requires formulation or re-constitution to create a bio-available material.

Chromatographic media can be used to capture and purify small molecules, vaccines, and biological species, for example. Many types of chromatographic media can be used in a positive capture mode.

US2010178210 discloses compositions and methods for storage of biomolecules, wherein the biomolecules are stored via absorption to a substrate. Absorbed biomolecules can be eluted or recovered from the substrate at a future time, and optionally be subjected to a subsequent analysis or application.

US2009029438 discloses a composite material that comprises a support member that has a plurality of pores extending through the support member and, located in the pores of the support member, and filling the pores of the support member, a macroporous cross-linked gel.

There exists a need for a method of stabilizing and storing pre-measured quantities of these therapeutic agents that optimizes the shelf-life, ease of transport, and thermal stability of the material. Furthermore, there exists a need for these storage devices to be streamlined with the ultimate delivery system, for example, intravenous drips or syringes.

## SUMMARY OF THE INVENTION

In a first aspect is provided a dried, stabilised composite material, comprising:

a support member, comprising a plurality of pores extending through the support member; and
a macroporous cross-linked gel, comprising a plurality of macropores;
a protein covalently bonded to the microporous cross-linked gel, wherein the protein is protein A; and
a stabilising agent comprising polyethylene glycol, glucose, glycerol or sucrose;
wherein the macroporous cross-linked gel is located in the pores of the support member; and the average pore diameter of the macropores is less than the average pore diameter of the pores.

In certain embodiments, the invention relates to any one of the aforementioned composite materials, wherein the composite material further comprises a therapeutic agent.

In certain embodiments, the invention relates to any one of the aforementioned composite materials, wherein the therapeutic agent is unstable. In certain embodiments, the invention relates to any one of the aforementioned composite materials, wherein the therapeutic agent is thermally unstable.

In certain embodiments, the invention relates to any one of the aforementioned composite materials, wherein the therapeutic agent is an antibody, a protein, or a virus. In certain embodiments, the invention relates to any one of the aforementioned composite materials, wherein the therapeutic agent is an antibody. In certain embodiments, the invention relates to any one of the aforementioned composite materials, wherein the therapeutic agent is a virus. In certain embodiments, the invention relates to any one of the aforementioned composite materials, wherein the therapeutic agent is a live virus. In certain embodiments, the invention relates to any one of the aforementioned composite materials, wherein the therapeutic agent is an oncolytic virus.

In certain embodiments, the invention relates to any one of the aforementioned composite materials, wherein the therapeutic agent is IgG.

In certain embodiments, the invention relates to any one of the aforementioned composite materials, wherein the therapeutic agent is 0547659 (Pfizer), agalsidase beta, alemtuzumab, alglucosidase alfa, alteplase, ALXN6000 (Dyax), AMG 386 (Dyax), AMG 479 (Dyax), AMG 780 (Dyax), AMG 888 (Daiikio Sankyo), anrukinzumab, anthrax vaccine, anti-CD19 MAb, anti-HB-EGF antibody, antihemophilic factor, anti-HER3 antibody, ASG-5ME (Seattle Genetics), ASG-22ME (Seattle Genetics), AV-203 (Aveo), bapineuzumab, BAY 94-9343 (Immunogen), bevacizumab, BI-204 (Dyax), BI-505 (Dyax), BIIB 033 (Dyax), *Bordetella pertussis* (inactivated), bortezomib, brentuximab vodetin, capecitabine, CDX-0011 (Celldex), CDX-014 (Celldex), CDX-301 (Celldex), CDX-1127 (Celldex), CDX-1135 (Celldex), CDX-1402 (Celldex), certolizumab pegol, cetuximab, cholera vaccine (WC-rBS), choriogonadotripin alfa, choriogonadotropin alfa (recombinant), cixutumumab, clofarabine, collagenase clostridium histolyticum, CT-322 (Bristol-Myers Squibb), DA-3801 (Dong-A), daclizumab, darbepoetin alfa, denosumab, diphtheria toxoid, doripenem, dornase alfa, ecallantide, eculizumab, enfuvirtide, eplerenone, epoetin alfa, erlotinib, ertapenem, erythropoietin (recombinant), etanercept, ficlatuzumab, filgrastim (recombinant), follitropin alfa, fully human anthrax monoclonal antibody, G-CSF, golimumab, haemophilus b conjugate vaccine, haemophilus b conjugate vaccine (meningococcal protein conjugate), hepatitis A vaccine, hepatitis B surface antigen, hepatitis B vaccine (recombinant), Hib oligosaccharide (conjugated to $CRM_{197}$), human anthrax immunoglobulin, human follicle-stimulating hormone (recombinant), human papillomavirus vaccine (recombinant), ibandronate, IMC-3C5 (Dyax), IMC-11fb (Imclone), IMC-18F1 (Imclone), IMC-303 (Imclone), IMC-305 (Imclone), IMC-2007S (Imclone), IMC-RON8 (Imclone), IMGN529 (Immunogen), imiglucerase, infliximab, influenza virus vaccine, influenza virus vaccine (inactivated), influenza virus vaccine (quadrivalent, live attenuated), inotuzumab ozogamicin, interferon alfa-2a, interferon alfa-2a (recombinant), interferon alfa-2b (recombinant), interferon beta-la, interleukin-21, laronidase, lorvotuzumab mertansine, lutropin alfa, measles virus vaccine (live attenuated), MEDI-3250 (Medimmune), MEDI-551 (Medimmune), MM-121 (Dyax), moxetumomab pasudotox, MT201 (Dyax), mycophenolate mofetil, natalizumab, necitumamab, NEGF (Blueblood), omalizumab, palivizumab, panitumumab, pegfilgrastim, peginterferon alfa-2a, peginterferon alfa-2b, pegylated-interferon lambda, PF-0 se alfa (Pfizer), PF-04236921 (Pfizer), plerixafor, pneumococcal conjugate vaccine, pneumococcal vaccine polyvalent, protective antigen anthrax vaccine (recombinant), ramucirumab, ranibizumab, rituximab, romiplostim, rubella virus vaccine (live attenuated, Wistar RA27/3 strain), samalizumab, SAR566658 (Immunogen), SAR650984 (Immunogen), SGN-75 (Seattle Genetics), SGN-CD19A (Seattle Genetics), somatropin, somatropin (recombinant DNA), taliglucerase alfa, tanezumab, tenecteplase, tetanus toxoid, thrombin, thyrotropin alfa, tigatuzumab, tocilizumab, trastuzumab, trastuzumab emtansine, typhoid vaccine (live TY21a), U3-1287 (Daiikio Sankyo), U3-1565 (Daiikio Sankyo), ustekinumab, or vemurafenib.

In certain embodiments, the invention relates to any one of the aforementioned composite materials, wherein the therapeutic agent is a small molecule.

In certain embodiments, the invention relates to any one of the aforementioned composite materials, wherein the therapeutic agent is ceftriaxone, clonazepam, diazepam, fludarabine, flumazenil, naproxen, orlistat, oseltamivir phosphate, saquinavir mesylate, or valganciclovir.

In certain embodiments, the invention relates to any one of the aforementioned composite materials, wherein the therapeutic agent is covalently bonded to the macroporous cross-linked gel. In certain embodiments, the invention relates to any one of the aforementioned composite materials, wherein the therapeutic agent is adsorbed to or absorbed on the macroporous cross-linked gel. In certain embodiments, the invention relates to any one of the aforementioned composite materials, wherein the therapeutic agent is reversibly adsorbed to or reversibly adsorbed on the macroporous cross-linked gel.

The invention relates to any one of the aforementioned composite materials, wherein the composite material is substantially free of water.

The invention relates to any one of the aforementioned composite materials, wherein the composite material comprises a stabilizing agent comprising polyethylene glycol, glucose, glycerol or sucrose.

The invention relates to any one of the aforementioned composite materials, wherein the stabilizing agent comprises polyethylene glycol, glucose, glycerol or sucrose.

In certain embodiments, the invention relates to any one of the aforementioned composite materials, wherein the stabilizing agent is polyethylene glycol. In certain embodiments, the invention relates to any one of the aforementioned composite materials, wherein the stabilizing agent is poly(ethylene glycol). In certain embodiments, the invention relates to any one of the aforementioned composite materials, wherein the stabilizing agent is polyethylene glycol; and the number average molecular weight ($M_n$) polyethylene glycol is from about 300 to about 40,000. In certain embodiments, the invention relates to any one of the aforementioned composite materials, wherein the stabilizing agent is polyethylene glycol; and the number average molecular weight ($M_n$) of the polyethylene glycol is about 1000.

In certain embodiments, the invention relates to any one of the aforementioned composite materials, wherein the stabilizing agent is a monofunctional polyethylene glycol.

In certain embodiments, the invention relates to any one of the aforementioned composite materials, wherein the stabilizing agent is poly(ethylene glycol) methyl ether, polyethylene glycol mono methyl ether mesylate, methoxypolyethylene glycol amine, methoxypolyethylene glycol propionic acid, O-methyl-O'-succinylpolyethylene glycol, or tetraglycol.

In certain embodiments, the invention relates to any one of the aforementioned composite materials, wherein the stabilizing agent is a difunctional polyethylene glycol. In certain embodiments, the invention relates to any one of the aforementioned composite materials, wherein the stabilizing agent is O-(2-carboxyethyl)polyethylene glycol, O-(2-aminoethyl)polyethylene glycol, poly(ethylene glycol) dimethyl ether, poly(ethylene glycol) distearate, poly(ethylene glycol) bis(amine), $\alpha,\omega$-bis{2-[(3-carboxy-1-oxopropyl)amino]ethyl}polyethylene glycol, poly(ethylene glycol) bis(carboxymethyl) ether, poly(ethylene glycol) butyl ether, poly(ethylene glycol) tetrahydrofurfuryl ether, poly(ethylene glycol) bis(carboxymethyl) ether, poly(ethylene glycol) sorbitol hexaoleate, poly(ethylene glycol) diacrylamide, poly(ethylene glycol) diacrylate, tetra(ethylene glycol) diacrylate, di(ethylene glycol) dimethacrylate, poly(ethylene glycol) dithiol, tri(ethylene glycol) divinyl ether, or poly(ethylene glycol) diglycidyl ether.

In certain embodiments, the invention relates to any one of the aforementioned composite materials, wherein the stabilizing agent is a multi-arm polyethylene glycol. In certain embodiments, the invention relates to any one of the aforementioned composite materials, wherein the stabilizing agent is glycerol ethoxylate, 4-arm amine-terminated poly(ethylene oxide), or 4-arm hydoxy-terminated poly(ethylene oxide).

In certain embodiments, the invention relates to any one of the aforementioned composite materials, wherein the stabilizing agent is a polyethylene glycol copolymer. In certain embodiments, the invention relates to any one of the aforementioned composite materials, wherein the stabilizing agent is poly(ethylene glycol)-block-poly(propylene glycol)-block-poly(ethylene glycol), poly(ethylene glycol-ran-propylene glycol), poly(propylene glycol)-block-poly(ethylene glycol)-block-poly(propylene glycol) bis(2-aminopropyl ether), poly(ethylene glycol)-block-poly(propylene glycol)-block-poly(ethylene glycol), poly(ethylene glycol)-block-poly($\varepsilon$-caprolactone) methyl ether, poly(ethylene glycol)-block-polylactide methyl ether, or poly(ethylene glycol) 4-nonylphenyl 3-sulfopropyl ether potassium salt.

Disclosed herein is a method, comprising the steps of:

contacting a therapeutic agent with any one of the aforementioned composite materials, thereby forming a composite material with an associated therapeutic agent;
contacting the composite material with the associated therapeutic agent with a first solution, wherein the first solution comprises a stabilizing agent, thereby forming a stabilized composite material; and
substantially drying the stabilized composite material at a temperature for an amount of time, thereby substantially removing water from the stabilizing composite material.

Disclosed herein is a method of delivering a therapeutic agent to a subject in need thereof, comprising the step of:

contacting any one of the aforementioned composite materials with a second solution, thereby dissociating the therapeutic agent from the composite material and forming a third solution; and
delivering the third solution to the subject.

**BRIEF DESCRIPTION OF THE FIGURES**

**Figure 1** depicts the binding capacities of membranes as a function of soaking times in trehalose solution.
**Figure 2** depicts the binding capacities of membranes as a function of drying times.
**Figure 3** depicts the binding capacities of membranes as a function of the concentration of trehalose.
**Figure 4** tabulates the binding capacities of membranes as a function of the concentration of buffer in a trehalose solution.
**Figure 5** tabulates the binding capacities of membranes as a function of various variables.
**Figure 6** tabulates the calculated mass of trehalose in the dried membranes.
**Figure 7** tabulates the binding capacity and flux over time of ProA-functionalized membranes stored at room temperature (top) and at 50 °C (bottom).
**Figure 8** depicts images at 400x (left) and 1200x (right) of the surface of a ProA-functionalized membrane after being stored at 50 °C for one week.
**Figure 9** tabulates the binding capacity over time of aldehyde-functionalized membranes dried at room temperature, and then stored at room temperature or at 50 °C.
**Figure 10** tabulates the flux over time of aldehyde-functionalized membranes dried at room temperature, and then stored at room temperature or at 50 °C.
**Figure 11** tabulates the binding capacity over time of aldehyde-functionalized membranes dried at 50 °C for 1 h, and then stored at room temperature or at 50 °C.
**Figure 12** tabulates the flux over time of aldehyde-functionalized membranes dried at 50 °C for 1 h, and then stored at room temperature or at 50 °C.
**Figure 13** tabulates the binding capacity over time of epoxy-functionalized membranes dried at room temperature, and then stored at room temperature or at 50 °C.
**Figure 14** tabulates the flux over time of epoxy-functionalized membranes dried at room temperature, and then stored at room temperature or at 50 °C.
**Figure 15** depicts the effect of flow rate of the eluent solution (IgG solution) on the binding capacity at 10% breakthrough (left) and at saturation (middle). Percent recovery is shown on the right. In each case, the left bar indicates 2 mL/min flow rate, and the right bar indicates 1 mL/min flow rate.
**Figure 16** depicts the effect of pH of the eluent solution on the binding capacity at 10% breakthrough (left) and at saturation (right). In each case, the left bar indicates pH 7.4, the middle bar indicates pH 6.5, and the right bar indicates pH 8.0.
**Figure 17** tabulates the binding capacity and percent recovery as a function of the identity of the buffer in the eluent solution.
**Figure 18** tabulates the binding capacity and percent recovery at various stages of breakthrough for an eluent solution of 0.2 M glycine + 0.2 M glucose and ethanol (8:2, v:v).
**Figure 19** tabulates the binding capacity and percent recovery for various eluent solutions containing glycine and NaCl.
**Figure 20** depicts percent recovery as a function of the pH of the eluent solution (left bar = pH 3.0, middle bar = pH 3.5, right bar = pH 4.0).
**Figure 21** tabulates the effect on the size of the membrane of storing various membranes at 50 °C.
**Figure 22** tabulates the effect on the size of the membrane of the ProA coupling process.
**Figure 23** depicts an ESEM image at 190x of the surface of a ProA-functionalized membrane that had been wrapped around a cylinder.

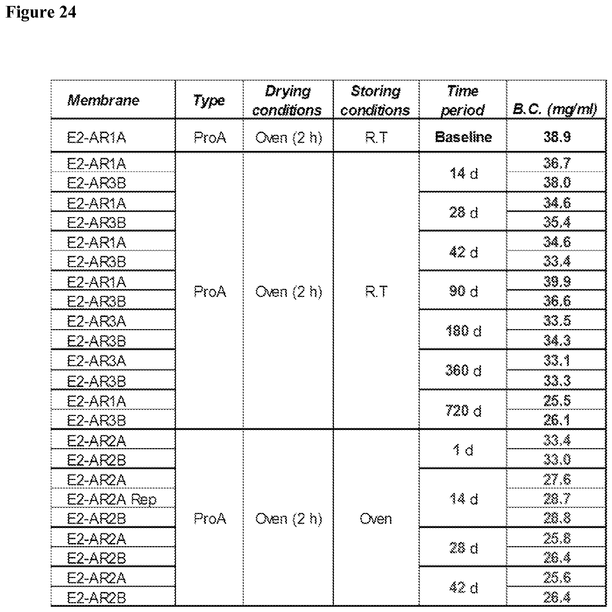

**Figure 24** tabulates the binding capacity over time of ProA-functionalized membranes stored at room temperature or at 50 °C.

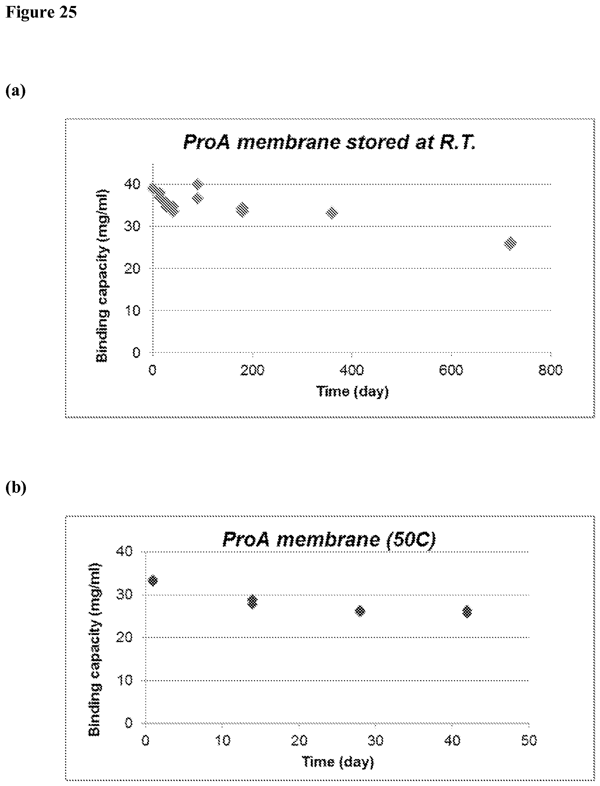

**Figure 25** depicts the binding capacity over time of ProA-functionalized membranes stored at (a) room temperature, or (b) 50 °C.

**Figure 26** tabulates the results from binding IgG to a composite material in the presence or absence of a stabilizing agent, drying the composite material, and then eluting IgG from the composite material after either 1 day or 16 days of storage at at 2-8 °C. **PEG1000 is polyethylene glycol with $M_n$ = 1000.

**Figure 27** tabulates the percentage of active IgG recovered after binding IgG to a composite material in the presence or absence of a stabilizing agent, drying the composite material, and then eluting IgG from the composite material after either 1 day or 16 days of storage at 2-8 °C.

**Figure 28** depicts an exemplary calibration curve for IgG concentration in a solution.

**Figure 29** tabulates the quantity of IgG bound to the composite material, the % of that IgG that was later eluted from the composite material by a buffer with pH 7.2, and the binding capacity of the composite material.

## DETAILED DESCRIPTION OF THE INVENTION

### *Overview*

There is a need for convenient storage and delivery media for unstable drugs and biologics. Many of the limitations of current methods can be minimized by using membranes and membrane processes. In certain embodiments, membrane separation processes are well-suited for large-scale applications because they combine the following attractive features: low-energy consumption, large processing capacity, low cost, high efficiency, simplicity, continuous operation mode, easy adaptation to a range of production-relevant process configurations, convenient up-scaling, high flux, and, in most cases, processing at ambient temperature.

In certain embodiments, the invention relates to a composite material comprising a macroporous gel within a porous support member. The composite materials are suited for the storage of unstable solutes, such as small molecules or biologics.

In certain embodiments, the invention relates to a method of reversible adsorption of a substance on a macroporous gel of a composite material.

In certain embodiments, an adsorbed substance may be released by allowing liquid to flow through the macroporous gel of the composite material. In certain embodiments, the uptake and release of substances may be controlled by variations in the composition of the macroporous cross-linked gel.

*Various Characteristics of Exemplary Composite Materials*

Composition of the Macroporous Gels

In certain embodiments, the macroporous gels may be formed through the *in situ* reaction of one or more polymerizable monomers with one or more cross-linkers. In certain embodiments, the macroporous gels may be formed through the reaction of one or more cross-linkable polymers with one or more cross-linkers. In certain embodiments, a cross-linked gel having macropores of a suitable size may be formed.

In certain embodiments, suitable polymerizable monomers include monomers containing vinyl or acryl groups. In certain embodiments, a polymerizable monomer is selected from the group consisting of acrylamide, N-acryloxysuccinimide, butyl acrylate and methacrylate, N,N-diethylacrylamide, N,N-dimethylacrylamide, 2-(N,N-dimethylamino)ethyl acrylate and methacrylate, N-[3-(N,N-dimethylamino)propyl]methacrylamide, N,N-dimethylacrylamide, n-dodecyl acrylate, n-dodecyl methacrylate, phenyl acrylate and methacrylate, dodecyl methacrylamide, ethyl acrylate and methacrylate, 2-ethylhexyl methacrylate, hydroxypropyl methacrylate, glycidyl acrylate and methacrylate, ethylene glycol phenyl ether methacrylate, n-heptyl acrylate and methacrylate, 1-hexadecyl acrylate and methacrylate, methacrylamide, methacrylic anhydride, octadecyl acrylamide, octylacrylamide, octyl methacrylate, propyl acrylate and methacrylate, N-iso-propylacrylamide, stearyl acrylate and methacrylate, styrene, alkylated styrene derivatives, 4-vinylpyridine, vinylsulfonic acid, and N-vinyl-2-pyrrolidinone (VP). In certain embodiments, the polymerizable monomers may comprise butyl, hexyl, phenyl, ether, or poly(propylene glycol) side chains. In certain embodiments, various other vinyl or acryl monomers comprising a reactive functional group may be used; these reactive monomers may be subsequently functionalized.

In certain embodiments, the monomer may comprise a reactive functional group. In certain embodiments, the reactive functional group of the monomer may be reacted with any of a variety of specific ligands. In certain embodiments, this technique allows for partial or complete control of ligand density or pore size. In certain embodiments, the reactive functional group of the monomer may be functionalized prior to the gel-forming reaction. In certain embodiments, the reactive functional group of the monomer may be functionalized subsequent to the gel-forming reaction. In certain embodiments, monomers, such as glycidyl methacrylate, acrylamidoxime, acrylic anhydride, azelaic anhydride, maleic anhydride, hydrazide, acryloyl chloride, 2-bromoethyl methacrylate, or vinyl methyl ketone, may be further functionalized.

In certain embodiments, the cross-linking agent may be a compound containing at least two vinyl or acryl groups. In certain embodiments, the cross-linking agent is selected from the group consisting of bisacrylamidoacetic acid, 2,2-bis[4-(2-acryloxyethoxy)phenyl]propane, 2,2-bis(4-methacryloxyphenyl)propane, butanediol diacrylate and dimethacrylate, 1,4-butanediol divinyl ether, 1,4-cyclohexanediol diacrylate and dimethacrylate, 1,10-dodecanediol diacrylate and dimethacrylate, 1,4-diacryloylpiperazine, diallylphthalate, 2,2-dimethylpropanediol diacrylate and dimethacrylate, dipentaerythritol pentaacrylate, dipropylene glycol diacrylate and dimethacrylate, N,N-dodecamethylenebisacrylamide, divinylbenzene, glycerol trimethacrylate, glycerol tris(acryloxypropyl) ether, N,N'-hexamethylenebisacrylamide, N,N'-octamethylenebisacrylamide, 1,5-pentanediol diacrylate and dimethacrylate, 1,3-phenylenediacrylate, poly(ethylene glycol) diacrylate and dimethacrylate, poly(propylene) diacrylate and dimethacrylate, triethylene glycol diacrylate and dimethacrylate, triethylene glycol divinyl ether, tripropylene glycol diacrylate or dimethacrylate, diallyl diglycol carbonate, poly(ethylene glycol) divinyl ether, N,N'-dimethacryloylpiperazine, divinyl glycol, ethylene glycol diacrylate, ethylene glycol dimethacrylate, N,N'-methylenebisacrylamide, 1,1,1-trimethylolethane trimethacrylate, 1,1,1-trimethylolpropane triacrylate, 1,1,1-trimethylolpropane trimethacrylate (TRIM-M), vinyl acrylate, 1,6-hexanediol diacrylate and dimethacrylate, 1,3-butylene glycol diacrylate and dimethacrylate, alkoxylated cyclohexane dimethanol diacrylate, alkoxylated hexanediol diacrylate, alkoxylated neopentyl glycol diacrylate, aromatic dimethacrylate, caprolactone modified neopentylglycol hydroxypivalate diacrylate, cyclohexane dimethanol diacrylate and dimethacrylate, ethoxylated bisphenol diacrylate and dimethacrylate, neopentyl glycol diacrylate and dimethacrylate, ethoxylated trimethylolpropane triacrylate, propoxylated trimethylolpropane triacrylate, propoxylated glyceryl triacrylate, pentaerythritol triacrylate, tris (2-hydroxy ethyl)isocyanurate triacrylate, di-trimethylolpropane tetraacrylate, dipentaerythritol pentaacrylate, ethoxylated pentaerythritol tetraacrylate, pentaacrylate ester, pentaerythritol tetraacrylate, caprolactone modified dipentaerythritol hexaacrylate, N,N',-methylenebisacrylamide, diethylene glycol diacrylate and dimethacrylate, trimethylolpropane triacrylate, ethylene glycol diacrylate and dimethacrylate, tetra(ethylene glycol) diacrylate, 1,6-hexanediol diacrylate, divinylbenzene, and poly(ethylene glycol) diacrylate.

In certain embodiments, the size of the macropores in the resulting gel increases as the concentration of cross-linking agent is increased. In certain embodiments, the mole percent (mol%) of cross-linking agent to monomer(s) may be about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, or about 60%.

In certain embodiments, the properties of the composite materials may be tuned by adjusting the average pore diameter of the macroporous gel. The size of the macropores is generally dependent on the nature and concentration of the cross-linking agent, the nature of the solvent or solvents in which the gel is formed, the amount of any polymerization initiator or catalyst and, if present, the nature and concentration of porogen. In certain embodiments, the composite material may have a narrow pore-size distribution.

Porous Support Member

In some embodiments, the porous support member is made of polymeric material and contains pores of average size between about 0.1 and about 25 $\mu$m, and a volume porosity between about 40% and about 90%. Many porous substrates or membranes can be used as the support member but the support may be a polymeric material. In certain embodiments, the support may be a polyolefin, which is available at low cost. In certain embodiments, the polyolefin may be poly(ethylene), poly(propylene), or poly(vinylidene difluoride). Extended polyolefin membranes made by thermally induced phase separation (TIPS) or non-solvent induced phase separation are mentioned. In certain embodiments, the support member may be made from natural polymers, such as cellulose or its derivatives. In certain embodiments, suitable supports include polyethersulfone membranes, poly(tetrafluoroethylene) membranes, nylon membranes, cellulose ester membranes, or filter papers.

In certain embodiments, the porous support is composed of woven or non-woven fibrous material, for example, a polyolefin, such as polypropylene. Such fibrous woven or non-woven support members can have pore sizes larger than the TIPS support members, in some instances up to about 75 $\mu$m. The larger pores in the support member permit formation of composite materials having larger macropores in the macroporous gel. Non-polymeric support members can also be used, such as ceramic-based supports. In certain embodiments, the support member is fiberglass. The porous support member can take various shapes and sizes.

In some embodiments, the support member is in the form of a membrane that has a thickness from about 10 to about 2000 $\mu$m, from about 10 to about 1000 $\mu$m, or from about 10 to about 500 $\mu$m. In other embodiments, multiple porous support units can be combined, for example, by stacking. In one embodiment, a stack of porous support membranes, for example, from 2 to 10 membranes, can be assembled before the macroporous gel is formed within the void of the porous support. In another embodiment, single support member units are used to form composite material membranes, which are then stacked before use.

Relationship Between Macroporous Gel and Support Member

The macroporous gel may be anchored within the support member. The term "anchored" is intended to mean that the gel is held within the pores of the support member, but the term is not necessarily restricted to mean that the gel is chemically bound to the pores of the support member. The gel can be held by the physical constraint imposed upon it by enmeshing and intertwining with structural elements of the support member, without actually being chemically grafted to the support member, although in some embodiments, the macroporous gel may be grafted to the surface of the pores of the support member.

Because the macropores are present in the gel that occupies the pores of the support member, the macropores of the gel must be smaller than the pores of the support member. Consequently, the flow characteristics and separation characteristics of the composite material are dependent on the characteristics of the macroporous gel, but are largely independent of the characteristics of the porous support member, with the proviso that the size of the pores present in the support member is greater than the size of the macropores of the gel. The porosity of the composite material can be tailored by filling the support member with a gel whose porosity is partially or completely dictated by the nature and amounts of monomer or polymer, cross-linking agent, reaction solvent, and any porogen, if used. As pores of the support member are filled with the same macroporous gel material, a high degree of consistency is achieved in properties of the composite material, and for a particular support member these properties are determined partially, if not entirely, by the properties of the macroporous gel. The net result is that the invention provides control over macropore size, permeability and surface area of the composite materials.

The number of macropores in the composite material is not dictated by the number of pores in the support material. The number of macropores in the composite material can be much greater than the number of pores in the support member because the macropores are smaller than the pores in the support member. As mentioned above, the effect of the pore-size of the support material on the pore-size of the macroporous gel is generally negligible. An exception is found in those cases where the support member has a large difference in pore-size and pore-size distribution, and where a macroporous gel having very small pore-sizes and a narrow range in pore-size distribution is sought. In these cases, large variations in the pore-size distribution of the support member are weakly reflected in the pore-size distribution of the macroporous gel. In certain embodiments, a support member with a somewhat narrow pore-size range may be used in these situations.

*Preparation of Composite Materials*

In certain embodiments, the composite materials of the invention may be prepared by single-step methods. In certain embodiments, these methods may use water or other environmentally benign solvents as the reaction solvent. In certain embodiments, the methods may be rapid and, therefore, may lead to easier manufacturing processes. In certain embodiments, preparation of the composite materials may be inexpensive.

In certain embodiments, the composite materials of the invention may be prepared by mixing one or more monomers, one or more cross-linking agents, one or more initiators, and optionally one or more porogens, in one or more suitable solvents. In certain embodiments, the resulting mixture may be homogeneous. In certain embodiments, the mixture may be heterogeneous. In certain embodiments, the mixture may then be introduced into a suitable porous support, where a gel forming reaction may take place.

In certain embodiments, suitable solvents for the gel-forming reaction include 1,3-butanediol, di(propylene glycol) propyl ether, N,N-dimethylacetamide, di(propylene glycol) methyl ether acetate (DPMA), water, dioxane, dimethylsulfoxide (DMSO), dimethylformamide (DMF), acetone, ethanol, N-methylpyrrolidone (NMP), tetrahydrofuran (THF), ethyl acetate, acetonitrile, toluene, xylenes, hexane, N-methylacetamide, propanol, methanol, or mixtures thereof. In certain embodiments, solvents that have a higher boiling point may be used, as these solvents reduce flammability and facilitate manufacture. In certain embodiments, solvents that have a low toxicity may be used, so they may be discarded, reused or recycled readily after use. An example of such a solvent is dipropyleneglycol monomethyl ether (DPM).

In certain embodiments, a porogen may be added to the reactant mixture, wherein porogens may be broadly described as pore-generating additives. In certain embodiments, the porogen is selected from the group consisting of poor solvents and extractable polymers, for example, poly(ethyleneglycol), surfactants, and salts.

In some embodiments, components of the gel forming reaction react spontaneously at room temperature to form the macroporous gel. In other embodiments, the gel forming reaction must be initiated. In certain embodiments, the gel forming reaction may be initiated by any known method, for example, through thermal activation or UV radiation. In certain embodiments, the reaction may be initiated by UV radiation in the presence of a photoinitiator. In certain embodiments, the photoinitiator is selected from the group consisting of 2-hydroxy-1-[4-2(hydroxyethoxy)phenyl]-2-methyl-1-propanone (Irgacure 2959), 2,2-dimethoxy-2-phenylacetophenone (DMPA), benzophenone, benzoin and benzoin ethers, such as benzoin ethyl ether and benzoin methyl ether, dialkoxyacetophenones, hydroxyalkylphenones, and α-hydroxymethyl benzoin sulfonic esters. Thermal activation may require the addition of a thermal initiator. In certain embodiments, the thermal initiator is selected from the group consisting of 1,1'-azobis(cyclohexanecarbonitrile) (VAZO® catalyst 88), azobis(isobutyronitrile) (AIBN), potassium persulfate, ammonium persulfate, and benzoyl peroxide.

In certain embodiments, the gel-forming reaction may be initiated by UV radiation. In certain embodiments, a photoinitiator may be added to the reactants of the gel forming reaction, and the support member containing the mixture of monomer, cross-linking agent, and photoinitiator may be exposed to UV radiation at wavelengths from about 250 nm to about 400 nm for a period of a few seconds to a few hours. In certain embodiments, the support member containing the mixture of monomer, cross-linking agent, and photoinitiator may be exposed to UV radiation at about 350 nm for a period of a few seconds to a few hours. In certain embodiments, the support member containing the mixture of monomer, cross-linking agent, and photoinitiator may be exposed to UV radiation at about 350 nm for about 10 minutes. In certain embodiments, visible wavelength light may be used to initiate the polymerization. In certain embodiments, the support member must have a low absorbance at the wavelength used so that the energy may be transmitted through the support member.

In certain embodiments, the rate at which polymerization is carried out may have an effect on the size of the macropores obtained in the macroporous gel. In certain embodiments, when the concentration of cross-linker in a gel is increased to sufficient concentration, the constituents of the gel begin to aggregate to produce regions of high polymer density and regions with little or no polymer, which latter regions are referred to as "macropores" in the present specification. This mechanism is affected by the rate of polymerization. In certain embodiments, the polymerization may be carried out slowly, such as when a low light intensity in the photopolymerization is used. In this instance, the aggregation of the gel constituents has more time to take place, which leads to larger pores in the gel. In certain embodiments, the polymerization may be carried out at a high rate, such as when a high intensity light source is used. In this instance, there may be less time available for aggregation and smaller pores are produced.

In certain embodiments, once the composite materials are prepared they may be washed with various solvents to remove any unreacted components and any polymer or oligomers that are not anchored within the support. In certain embodiments, solvents suitable for washing the composite material include water, acetone, methanol, ethanol, N,N-dimethylacetamide, pyridine, and DMF.

*Pore Size Determination*

*SEM and ESEM*

The average diameter of the macropores in the macroporous cross-linked gel may be estimated by one of many methods. One method that may be employed is scanning electron microscopy (SEM). SEM is a well-established method for determining pore sizes and porosities in general, and for characterizing membranes in particular. Reference is made to the book *Basic Principles of Membrane Technology* by Marcel Mulder (© 1996) ("Mulder"), especially Chapter IV. Mulder provides an overview of methods for characterizing membranes. For porous membranes, the first method mentioned is electron microscopy. SEM is a very simple and useful technique for characterising microfiltration membranes. A clear and concise picture of the membrane can be obtained in terms of the top layer, cross-section and bottom layer. In addition, the porosity and pore size distribution can be estimated from the photographs.

Environmental SEM (ESEM) is a technique that allows for the non-destructive imaging of specimens that are wet, by allowing for a gaseous environment in the specimen chamber. The environmental secondary detector (ESD) requires a gas background to function and operates at from about 3 torr to about 20 torr. These pressure restraints limit the ability to vary humidity in the sample chamber. For example, at 10 torr, the relative humidity at a specific temperature is as follows:

| Relative Humidity at 10 torr (%) | T (°C) |
|---|---|
| About 80 | About 16 |
| About 70 | About 18 |
| About 60 | About 20 |
| About 40 | About 24 |
| About 20 | About 40 |
| About 10 | About 50 |
| About 2 | About 70 |
| About 1 | About 100 |

This is a useful guide to relative humidity in the sample chamber at different temperatures. In certain embodiments, the relative humidity in the sample chamber during imaging is from about 1% to about 99%. In certain embodiments, the relative humidity in the sample chamber during imaging is about 1%, about 2%, about 3%, about 4%, about 5%, about 6%, about 7%, about 8%, about 9%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 99%. In certain embodiments, the relative humidity in the sample chamber during imaging is about 45 %

In certain embodiments, the microscope has nanometer resolution and up to about 100,000X magnification.

In certain embodiments, the temperature in the sample chamber during imaging is from about 1 °C to about 95 °C. In certain embodiments, the temperature in the sample chamber during imaging is about 2 °C, about 3 °C, about 4 °C, about 5 °C, about 6 °C, about 7 °C, about 8 °C, about 9 °C, about 10 °C, about 12 °C, about 14 °C, about 16 °C, about 18 °C, about 20 °C, about 25 °C, about 30 °C, about 35 °C, about 40 °C, about 45 °C, about 50 °C, about 55 °C, about 60 °C, about 65 °C, about 70 °C, about 75 °C, about 80 °C, or about 85 °C. In certain embodiments, the temperature in the sample chamber during imaging is about 5 °C

In certain embodiments, the pressure in the sample chamber during imaging is from about 0.5 torr to about 20 torr. In certain embodiments, the pressure in the sample chamber during imaging is about 4 torr, about 6 torr, about 8 torr, about 10 torr, about 12 torr, about 14 torr, about 16 torr, about 18 torr, or about 20 torr. In certain embodiments, the pressure in the sample chamber during imaging is about 3 torr.

In certain embodiments, the working distance from the source of the electron beam to the sample is from about 6 mm to about 15 mm. In certain embodiments, the working distance from the source of the electron beam to the sample is about 6 mm, about 7 mm, about 8 mm, about 9 mm, about 10 mm, about 11 mm, about 12 mm, about 13 mm, about 14 mm, or about 15 mm. In certain embodiments, the working distance from the source of the electron beam to the sample is about 10 mm.

In certain embodiments, the voltage is from about 1 kV to about 30 kV. In certain embodiments, the voltage is about 2 kV, about 4 kV, about 6 kV, about 8 kV, about 10 kV, about 12 kV, about 14 kV, about 16 kV, about 18 kV, about 20 kV, about 22 kV, about 24 kV, about 26 kV, about 28 kV, or about 30 kV. In certain embodiments, the voltage is about 20 kV.

In certain embodiments, the average pore diameter may be measured by estimating the pore diameters in a representative sample of images from the top or bottom of a composite material. One of ordinary skill in the art will recognize and acknowledge various experimental variables associated with obtaining an ESEM image of a wetted membrane, and will be able to design an experiment accordingly.

*Capillary Flow Porometry*

Capillary flow porometry is an analytical technique used to measure the pore size(s) of porous materials. In this analytical technique, a wetting liquid is used to fill the pores of a test sample and the pressure of a non-reacting gas is used to displace the liquid from the pores. The gas pressure and flow rate through the sample is accurately measured and the pore diameters are determined using the following equation: The gas pressure required to remove liquid from the pores is related to the size of the pore by the following equation:

$$D = 4 \times \gamma \times \cos\theta / P$$

D = pore diameter
γ = liquid surface tension
θ = liquid contact angle
P = differential gas pressure

This equation shows that the pressure required to displace liquid from the wetted sample is inversely related to the pore size. Since this technique involves the flow of a liquid from the pores of the test sample under pressure, it is useful for the characterization of "through pores" (interconnected pores that allow fluid flow from one side of the sample to the other). Other pore types (closed and blind pores) are not detectable by this method.

Capillary flow porometry detects the presence of a pore when gas starts flowing through that pore. This occurs only when the gas pressure is high enough to displace the liquid from the most constricted part of the pore. Therefore, the pore diameter calculated using this method is the diameter of the pore at the most constricted part and each pore is detected as a single pore of this constricted diameter. The largest pore diameter (called the bubble point) is determined by the lowest gas pressure needed to initiate flow through a wet sample and a mean pore diameter is calculated from the mean flow pressure. In addition, both the constricted pore diameter range and pore size distribution may be determined using this technique.

This method may be performed on small membrane samples (~e.g., about 2.5 cm diameter) that are immersed in a test fluid (e.g. water, buffer, alcohol). The range of gas pressure applied can be selected from about 0 to about 500 psi.

*Other Methods of Determining Pore Diameter*

Mulder describes other methods of characterizing the average pore size of a porous membrane, including atomic force microscopy (AFM) (page 164), permeability calculations (page 169), gas adsorption-desorption (page 173), thermoporometry (page 176), permporometry (page 179), and liquid displacement (page 181).

*Exemplary Composite Materials*

In certain embodiments, composite materials have been previously described, for example, in U.S. Patent No. 7,316,919, and U.S. Patent Application Publication Nos. 2008/0314831, 2008/0312416, 2009/0029438, 2009/0032463, 2009/0008328, 2009/0035552, 2010/0047551, 2010/0044316, 2008/0017578, and 2011/0253616.

The invention relates to a dried, stabilised composite material, comprising:

a support member, comprising a plurality of pores extending through the support member; and
a macroporous cross-linked gel, comprising a plurality of macropores;
a protein covalently bonded to the microporous cross-linked gel, wherein the protein is protein A; and
a stabilising agent comprising polyethylene glycol, glucose, glycerol or sucrose;
wherein the macroporous cross-linked gel is located in the pores of the support member; and the average pore diameter of the macropores is less than the average pore diameter of the pores.

In certain embodiments, the invention relates to any one of the aforementioned composite materials, wherein the composite material further comprises a therapeutic agent.

In certain embodiments, the invention relates to any one of the aforementioned composite materials, wherein the therapeutic agent is unstable. In certain embodiments, the invention relates to any one of the aforementioned composite materials, wherein the therapeutic agent is thermally unstable.

In certain embodiments, the invention relates to any one of the aforementioned composite materials, wherein the therapeutic agent is an antibody, a protein, or a virus. In certain embodiments, the invention relates to any one of the aforementioned composite materials, wherein the therapeutic agent is an antibody. In certain embodiments, the invention relates to any one of the aforementioned composite materials, wherein the therapeutic agent is a virus. In certain embodiments, the invention relates to any one of the aforementioned composite materials, wherein the therapeutic agent is a live virus. In certain embodiments, the invention relates to any one of the aforementioned composite materials, wherein the therapeutic agent is an oncolytic virus.

In certain embodiments, the invention relates to any one of the aforementioned composite materials, wherein the therapeutic agent is IgG.

In certain embodiments, the invention relates to any one of the aforementioned composite materials, wherein the therapeutic agent is 0547659 (Pfizer), agalsidase beta, alemtuzumab, alglucosidase alfa, alteplase, ALXN6000 (Dyax), AMG 386 (Dyax), AMG 479 (Dyax), AMG 780 (Dyax), AMG 888 (Daiikio Sankyo), anrukinzumab, anthrax vaccine, anti-CD19 MAb, anti-HB-EGF antibody, antihemophilic factor, anti-HER3 antibody, ASG-5ME (Seattle Genetics), ASG-22ME (Seattle Genetics), AV-203 (Aveo), bapineuzumab, BAY 94-9343 (Immunogen), bevacizumab, BI-204 (Dyax), BI-505 (Dyax), BIIB 033 (Dyax), *Bordetella pertussis* (inactivated), bortezomib, brentuximab vodetin, capecitabine, CDX-0011 (Celldex), CDX-014 (Celldex), CDX-301 (Celldex), CDX-1127 (Celldex), CDX-1135 (Celldex), CDX-1402 (Celldex), certolizumab pegol, cetuximab, cholera vaccine (WC-rBS), choriogonadotripin alfa, choriogonadotropin alfa (recombinant), cixutumumab, clofarabine, collagenase clostridium histolyticum, CT-322 (Bristol-Myers Squibb), DA-3801 (Dong-A), daclizumab, darbepoetin alfa, denosumab, diphtheria toxoid, doripenem, dornase alfa, ecallantide, eculizumab, enfuvirtide, eplerenone, epoetin alfa, erlotinib, ertapenem, erythropoietin (recombinant), etanercept, ficlatuzumab, filgrastim (recombinant), follitropin alfa, fully human anthrax monoclonal antibody, G-CSF, golimumab, haemophilus b conjugate vaccine, haemophilus b conjugate vaccine (meningococcal protein conjugate), hepatitis A vaccine, hepatitis B surface antigen, hepatitis B vaccine (recombinant), Hib oligosaccharide (conjugated to $CRM_{197}$), human anthrax immunoglobulin, human follicle-stimulating hormone (recombinant), human papillomavirus vaccine (recombinant), ibandronate, IMC-3C5 (Dyax), IMC-11fb (Imclone), IMC-18F1 (Imclone), IMC-303 (Imclone), IMC-305 (Imclone), IMC-2007S (Imclone), IMC-RON8 (Imclone), IMGN529 (Immunogen), imiglucerase, infliximab, influenza virus vaccine, influenza virus vaccine (inactivated), influenza virus vaccine (quadrivalent, live attenuated), inotuzumab ozogamicin, interferon alfa-2a, interferon alfa-2a (recombinant), interferon alfa-2b (recombinant), interferon beta-la, interleukin-21, laronidase, lorvotuzumab mertansine, lutropin alfa, measles virus vaccine (live attenuated), MEDI-3250 (Medimmune), MEDI-551 (Medimmune), MM-121 (Dyax), moxetumomab pasudotox, MT201 (Dyax), mycophenolate mofetil, natalizumab, necitumamab, NEGF (Blueblood), omalizumab, palivizumab, panitumumab, pegfilgrastim, peginterferon alfa-2a, peginterferon alfa-2b, pegylated-interferon lambda, PF-0 se alfa (Pfizer), PF-04236921 (Pfizer), plerixafor, pneumococcal conjugate vaccine, pneumococcal vaccine polyvalent, protective antigen anthrax vaccine (recombinant), ramucirumab, ranibizumab, rituximab, romiplostim, rubella virus vaccine (live attenuated, Wistar RA27/3 strain), samalizumab, SAR566658 (Immunogen), SAR650984 (Immunogen), SGN-75 (Seattle Genetics), SGN-CD19A (Seattle Genetics), somatropin, somatropin (recombinant DNA), taliglucerase alfa, tanezumab, tenecteplase, tetanus toxoid, thrombin, thyrotropin alfa, tigatuzumab, tocilizumab, trastuzumab, trastuzumab emtansine, typhoid vaccine (live TY21a), U3-1287 (Daiikio Sankyo), U3-1565 (Daiikio Sankyo), ustekinumab, or vemurafenib.

In certain embodiments, the invention relates to any one of the aforementioned composite materials, wherein the therapeutic agent is a small molecule.

In certain embodiments, the invention relates to any one of the aforementioned composite materials, wherein the therapeutic agent is ceftriaxone, clonazepam, diazepam, fludarabine, flumazenil, naproxen, orlistat, oseltamivir phosphate, saquinavir mesylate, or valganciclovir.

In certain embodiments, the invention relates to any one of the aforementioned composite materials, wherein the therapeutic agent is covalently bonded to the macroporous cross-linked gel. In certain embodiments, the invention relates to any one of the aforementioned composite materials, wherein the therapeutic agent is adsorbed to or absorbed on the macroporous cross-linked gel. In certain embodiments, the invention relates to any one of the aforementioned composite materials, wherein the therapeutic agent is reversibly adsorbed to or reversibly adsorbed on the macroporous cross-linked gel.

The invention relates to any one of the aforementioned composite materials, wherein the composite material is substantially free of water.

The invention relates to any one of the aforementioned composite materials, wherein the composite material comprises a stabilizing agent comprising polyethylene glycol, glucose, glycerol or sucrose. The invention relates to any one of the aforementioned composite materials, wherein the stabilizing agent comprises polyethylene glycol, glucose, glycerol or sucrose.

In certain embodiments, the invention relates to any one of the aforementioned composite materials, wherein the stabilizing agent is polyethylene glycol. In certain embodiments, the invention relates to any one of the aforementioned composite materials, wherein the stabilizing agent is poly(ethylene glycol). In certain embodiments, the invention relates to any one of the aforementioned composite materials, wherein the stabilizing agent is polyethylene glycol; and the number average molecular weight ($M_n$) of the polyethylene glycol is from about 300 to about 40,000. In certain embodiments, the invention relates to any one of the aforementioned composite materials, wherein the stabilizing agent is polyethylene glycol; and the number average molecular weight ($M_n$) of the polyethylene glycol is about 1000.

In certain embodiments, the invention relates to any one of the aforementioned composite materials, wherein the stabilizing agent is a monofunctional polyethylene glycol.

In certain embodiments, the invention relates to any one of the aforementioned composite materials, wherein the stabilizing agent is poly(ethylene glycol) methyl ether, polyethylene glycol mono methyl ether mesylate, methoxypolyethylene glycol amine, methoxypolyethylene glycol propionic acid, O-methyl-O'-succinylpolyethylene glycol, or tetraglycol.

In certain embodiments, the invention relates to any one of the aforementioned composite materials, wherein the stabilizing agent is a difunctional polyethylene glycol. In certain embodiments, the invention relates to any one of the aforementioned composite materials, wherein the stabilizing agent is O-(2-carboxyethyl)polyethylene glycol, O-(2-aminoethyl)polyethylene glycol, poly(ethylene glycol) dimethyl ether, poly(ethylene glycol) distearate, poly(ethylene glycol) bis(amine), $\alpha,\omega$-bis{2-[(3-carboxy-1-oxopropyl)amino]ethyl}polyethylene glycol, poly(ethylene glycol) bis(carboxymethyl) ether, poly(ethylene glycol) butyl ether, poly(ethylene glycol) tetrahydrofurfuryl ether, poly(ethylene glycol) bis(carboxymethyl) ether, poly(ethylene glycol) sorbitol hexaoleate, poly(ethylene glycol) diacrylamide, poly(ethylene glycol) diacrylate, tetra(ethylene glycol) diacrylate, di(ethylene glycol) dimethacrylate, poly(ethylene glycol) dithiol, tri(ethylene glycol) divinyl ether, or poly(ethylene glycol) diglycidyl ether.

In certain embodiments, the invention relates to any one of the aforementioned composite materials, wherein the stabilizing agent is a multi-arm polyethylene glycol. In certain embodiments, the invention relates to any one of the aforementioned composite materials, wherein the stabilizing agent is glycerol ethoxylate, 4-arm amine-terminated poly(ethylene oxide), or 4-arm hydoxy-terminated poly(ethylene oxide).

In certain embodiments, the invention relates to any one of the aforementioned composite materials, wherein the stabilizing agent is a polyethylene glycol copolymer. In certain embodiments, the invention relates to any one of the aforementioned composite materials, wherein the stabilizing agent is poly(ethylene glycol)-block-poly(propylene glycol)-block-poly(ethylene glycol), poly(ethylene glycol-ran-propylene glycol), poly(propylene glycol)-block-poly(ethylene glycol)-block-poly(propylene glycol) bis(2-aminopropyl ether), poly(ethylene glycol)-block-poly(propylene glycol)-block-poly(ethylene glycol), poly(ethylene glycol)-block-poly($\varepsilon$-caprolactone) methyl ether, poly(ethylene glycol)-block-polylactide methyl ether, or poly(ethylene glycol) 4-nonylphenyl 3-sulfopropyl ether potassium salt.

In certain embodiments, the invention relates to any one of the aforementioned composite materials, wherein the stabilizing agent is poly(ethylene succinate), poly(ethylene adipate), or poly(ethylene-co-vinyl acetate).

In certain embodiments, the invention relates to any one of the aforementioned composite materials, wherein the composite material further comprises a salt.

In certain embodiments, the invention relates to any one of the aforementioned composite materials, wherein the salt is a phosphate salt. In certain embodiments, the invention relates to any one of the aforementioned composite materials, wherein the salt is ammonium acetate, ammonium formate, ammonium nitrate, ammonium phosphate, ammonium tartrate, potassium acetate, potassium citrate, potassium formate, potassium phosphate, sodium acetate, sodium formate, sodium phosphate, or sodium tartrate.

In certain embodiments, the invention relates to any one of the aforementioned composite materials, wherein the composite material is substantially stable at about 20 °C, about 25 °C, about 30 °C, about 35 °C, about 40 °C, about 45 °C, about 50 °C, about 55 °C, or about 60 °C.

In certain embodiments, the invention relates to any one of the aforementioned composite materials, wherein the composite material is substantially stable for about 7 d, about 14 d, about 21 d, about 28 d, about 35 d, about 42 d, about 49 d, about 75 d, about 100 d, about 125 d, about 150 d, about 175 d, about 200 d, about 225 d, about 250 d, about 275 d, about 300 d, about 325 d, about 400 d, about 425 d, about 450 d, about 475 d, about 500 d, about 525 d, about 550 d, about 575 d, about 600 d, about 625 d, about 650 d, about 675 d, about 700 d, about 725 d, about 750 d, about 775 d, or about 800 d.

In certain embodiments, the invention relates to any one of the aforementioned composite materials, wherein the composite material is substantially stable at about 50 °C for about 7 d, about 14 d, about 21 d, about 28 d, about 35 d, about 42 d, about 49 d, about 75 d, about 100 d, about 125 d, about 150 d, about 175 d, about 200 d, about 225 d, about 250 d, about 275 d, about 300 d, about 325 d, about 400 d, about 425 d, about 450 d, about 475 d, about 500 d, about 525 d, about 550 d, about 575 d, about 600 d, about 625 d, about 650 d, about 675 d, about 700 d, about 725 d, about 750 d, about 775 d, or about 800 d.

In certain embodiments, the invention relates to any one of the aforementioned composite materials, wherein "substantially stable" refers to a loss of binding capacity of less than about 40%, less than about 35%, less than about 30%, less than about 25%, less than about 20%, less than about 15%, or less than about 10% from the binding capacity at baseline (day zero).

In certain embodiments, the invention relates to any one of the aforementioned composite materials, wherein the mass ratio of the stabilizing agent to the therapeutic agent is about 10:1, about 9:1, about 8:1, about 7:1, about 6:1, about 5:1, about 4:1, about 3:1, about 2:1, about 1:1, about 1:2, or about 1:4.

In certain embodiments, the invention relates to any one of the aforementioned composite materials, wherein the quantity of therapeutic agent on the composite material represents a known quantity.

In certain embodiments, the invention relates to any one of the aforementioned composite materials, wherein the macroporous cross-linked gel comprises a polymer derived from acrylamide, N-acryloxysuccinimide, butyl acrylate or methacrylate, N,N-diethylacrylamide, N,N-dimethylacrylamide, 2-(N,N-dimethylamino)ethyl acrylate or methacrylate, 2-(N,N-diethylamino)ethyl acrylate or methacrylate N-[3-(N,N-dimethylamino)propyl]methacrylamide, N,N-dimethylacrylamide, n-dodecyl acrylate, n-dodecyl methacrylate, phenyl acrylate or methacrylate, dodecyl methacrylamide, ethyl acrylate or methacrylate, 2-ethylhexyl acrylate or methacrylate, hydroxypropyl acrylate or methacrylate, glycidyl acrylate or methacrylate, ethylene glycol phenyl ether methacrylate, n-heptyl acrylate or methacrylate, 1-hexadecyl acrylate or methacrylate, methacrylamide, methacrylic anhydride, octadecyl acrylamide, octylacrylamide, octyl acrylate or methacrylate, propyl acrylate or methacrylate, N-iso-propylacrylamide, stearyl acrylate or methacrylate, styrene, alkylated styrene derivatives, 4-vinylpyridine, vinylsulfonic acid, N-vinyl-2-pyrrolidinone (VP), acrylamido-2-methyl-1-propanesulfonic acid, styrenesulfonic acid, alginic acid, (3-acrylamidopropyl)trimethylammonium halide, diallyldimethylammonium halide, 4-vinyl-N-methylpyridinium halide, vinylbenzyl-N-trimethylammonium halide, methacryloxyethyltrimethylammonium halide, or 2-(2-methoxy)ethyl acrylate or methacrylate. In certain embodiments, the halide is chloride, bromide, or iodide.

In certain embodiments, the invention relates to any one of the aforementioned composite materials, wherein the macroporous cross-linked gel comprises a polymer derived from acrylamide, butyl acrylate or methacrylate, ethyl acrylate or methacrylate, 2-ethylhexyl methacrylate, hydroxypropyl acrylate or methacrylate, hydroxyethyl acrylate or methacrylate, hydroxymethyl acrylate or methacrylate, glycidyl acrylate or methacrylate, propyl acrylate or methacrylate, or N-vinyl-2-pyrrolidinone (VP).

In certain embodiments, the invention relates to any one of the aforementioned composite materials, wherein the macroporous cross-linked gel has a volume porosity from about 30% to about 80%; and the macropores have an average pore diameter from about 10 nm to about 3000 nm.

In certain embodiments, the invention relates to any one of the aforementioned composite materials, wherein the macroporous cross-linked gel has a volume porosity from about 40% to about 70%. In certain embodiments, the invention relates to any one of the aforementioned composite materials, wherein the macroporous cross-linked gel has a volume porosity of about 40%, about 45%, about 50%, about 55%, about 60%, about 65%, or about 70%.

In certain embodiments, the invention relates to any one of the aforementioned composite materials, wherein the average pore diameter of the macropores is about 25 nm to about 1000 nm.

In certain embodiments, the invention relates to any one of the aforementioned composite materials, wherein the average pore diameter of the macropores is about 50 nm to about 500 nm. In certain embodiments, the invention relates to any one of the aforementioned composite materials, wherein the average pore diameter of the macropores is about 50 nm, about 100 nm, about 150 nm, about 200 nm, about 250 nm, about 300 nm, about 350 nm, about 400 nm, about 450 nm, or about 500 nm.

In certain embodiments, the invention relates to any one of the aforementioned composite materials, wherein the average pore diameter of the macropores is from about 200 nm to about 300 nm. In certain embodiments, the invention relates to any one of the aforementioned composite materials, wherein the average pore diameter of the macropores is from about 75 nm to about 150 nm.

In certain embodiments, the invention relates to any one of the aforementioned composite materials, wherein the composite material is a membrane.

In certain embodiments, the invention relates to any one of the aforementioned composite materials, wherein the support member has a void volume; and the void volume of the support member is substantially filled with the macroporous cross-linked gel.

In certain embodiments, the invention relates to any one of the aforementioned composite materials, wherein the support member comprises a polymer; the support member is about 10 $\mu$m to about 5000 $\mu$m thick; the pores of the support member have an average pore diameter from about 0.1 $\mu$m to about 25 $\mu$m; and the support member has a volume porosity from about 40% to about 90%.

In certain embodiments, the invention relates to any one of the aforementioned composite materials, wherein the support member is about 10 $\mu$m to about 500 $\mu$m thick. In certain embodiments, the invention relates to any one of the aforementioned composite materials, wherein the support member is about 30 $\mu$m to about 300 $\mu$m thick. In certain embodiments, the invention relates to any one of the aforementioned composite materials, wherein the support member is about 30 $\mu$m, about 50 $\mu$m, about 100 $\mu$m, about 150 $\mu$m, about 200 $\mu$m, about 250 $\mu$m, or about 300 $\mu$m thick. In certain embodiments, the invention relates to any one of the aforementioned composite materials, wherein a plurality of support members from about 10 $\mu$m to about 500 $\mu$m thick may be stacked to form a support member up to about 5000 $\mu$m thick.

In certain embodiments, the invention relates to any one of the aforementioned composite materials, wherein the pores of the support member have an average pore diameter from about 0.1 $\mu$m to about 25 $\mu$m. In certain embodiments, the invention relates to any one of the aforementioned composite materials, wherein the pores of the support member have an average pore diameter from about 0.5 $\mu$m to about 15 $\mu$m. In certain embodiments, the invention relates to any one of the aforementioned composite materials, wherein the pores of the support member have an average pore diameter of about 0.5 $\mu$m, about 1 $\mu$m, about 2 $\mu$m, about 3 $\mu$m, about 4 $\mu$m, about 5 $\mu$m, about 6 $\mu$m, about 7 $\mu$m, about 8 $\mu$m, about 9 $\mu$m, about 10 $\mu$m, about 11 $\mu$m, about 12 $\mu$m, about 13 $\mu$m, about 14 $\mu$m, or about 15 $\mu$m.

In certain embodiments, the invention relates to any one of the aforementioned composite materials, wherein the support member has a volume porosity from about 40% to about 90%. In certain embodiments, the invention relates to any one of the aforementioned composite materials, wherein the support member has a volume porosity from about 50% to about 80%. In certain embodiments, the invention relates to any one of the aforementioned composite materials, wherein the support member has a volume porosity of about 50%, about 60%, about 70%, or about 80%.

In certain embodiments, the invention relates to any one of the aforementioned composite materials, wherein the support member comprises a polyolefin.

In certain embodiments, the invention relates to any one of the aforementioned composite materials, wherein the support member comprises a polymeric material selected from the group consisting of polysulfones, polyethersulfones, polyphenyleneoxides, polycarbonates, polyesters, cellulose and cellulose derivatives.

In certain embodiments, the invention relates to any one of the aforementioned composite materials, wherein the support member comprises a non-woven fiberglass.

In certain embodiments, the invention relates to any one of the aforementioned composite materials, wherein the support member comprises a fibrous woven or non-woven fabric comprising a polymer; the support member is from about 10 $\mu$m to about 2000 $\mu$m thick; the pores of the support member have an average pore diameter of from about 0.1 $\mu$m to about 25 $\mu$m; and the support member has a volume porosity from about 40% to about 90%.

In certain embodiments, the invention relates to any one of the aforementioned composite materials, wherein the support member comprises a non-woven material comprising fiberglass; the support member is from about 10 $\mu$m to about 5000 $\mu$m thick; the pores of the support member have an average pore diameter of from about 0.1 $\mu$m to about 50 $\mu$m; and the support member has a volume porosity from about 40% to about 90%.

*Exemplary Methods of the disclosure*

Disclosed herein is a method, comprising the steps of:

    contacting a therapeutic agent with any one of the aforementioned composite materials, thereby forming a composite material with an associated therapeutic agent;
    contacting the composite material with the associated therapeutic agent with a first solution, wherein the first solution comprises a stabilizing agent, thereby forming a stabilized composite material; and
    substantially drying the stabilized composite material at a temperature for an amount of time, thereby substantially removing water from the stabilizing composite material.

The disclosure relates to any one of the aforementioned methods, wherein the therapeutic agent is contacted with the composite material in the presence of a buffer salt. The disclosure relates to any one of the aforementioned methods, wherein the buffer salt comprises an acetate. The disclosure relates to any one of the aforementioned methods, wherein the buffer salt comprises sodium acetate. The disclosure relates to any one of the aforementioned methods, wherein the buffer salt is sodium acetate.

The disclosure relates to any one of the aforementioned methods, further comprising the step of washing the composite material after it has been contacted with the therapeutic agent. The disclosure relates to any one of the aforementioned methods, wherein the composite material is washed with a buffer. The disclosure relates to any one of the aforementioned methods, wherein the buffer comprises an acetate. The disclosure relates to any one of the aforementioned methods, wherein the buffer comprises sodium acetate. The disclosure relates to any one of the aforementioned methods, wherein the buffer is sodium acetate.

The disclosure relates to any one of the aforementioned methods, wherein the first solution further comprises a buffer salt. The disclosure relates to any one of the aforementioned methods, wherein the buffer salt comprises a phosphate or an acetate. The disclosure relates to any one of the aforementioned methods, wherein the buffer salt comprises an acetate. The disclosure relates to any one of the aforementioned methods, wherein the buffer salt comprises sodium acetate. The disclosure relates to any one of the aforementioned methods, wherein the buffer salt is sodium acetate. The disclosure relates to any one of the aforementioned methods, wherein the buffer salt is sodium acetate at about pH 5.

The disclosure relates to any one of the aforementioned methods, wherein the concentration of the stabilizing agent in the first solution is about 5 wt%, about 10 wt%, about 15 wt%, about 20 wt%, about 30 wt%, about 40 wt%, or about 50 wt%.

The disclosure relates to any one of the aforementioned methods, wherein the concentration of the buffer salt in the first solution is about 20 mM, about 30 mM, about 40 mM, about 50 mM, about 60 mM, about 70 mM, about 80 mM, about 90 mM, about 100 mM, about 110 mM, about 120 mM, about 130 mM, about 140 mM, about 150 mM, about 160 mM, about 170 mM, about 180 mM, about 190 mM, about 200 mM, about 210 mM, about 220 mM, about 230 mM, about 240 mM, or about 250 mM. The disclosure relates to any one of the aforementioned methods, wherein the concentration of the buffer salt in the first solution is about 85 mM.

The disclosure relates to any one of the aforementioned methods, wherein the pH of the first solution is about 4, about 4.5, about 5, about 5.5, about 6, about 6.5, about 7, about 7.5, or about 8. The disclosure relates to any one of the aforementioned methods, wherein the pH of the first solution is about 5.

The disclosure relates to any one of the aforementioned methods, wherein the composite material with the associated therapeutic agent is soaked in the first solution.

The disclosure relates to any one of the aforementioned methods, wherein the composite material with the associated therapeutic agent is contacted with the first solution for about 1 min., about 2 min., about 3 min., about 4 min., about 5 min., about 10 min., about 20 min., about 30 min., about 40 min., about 50 min., about 60 min., about 70 min, about 80 min., about 90 min., about 100 min., about 110 min., about 120 min., about 130 min., or about 140 min.

The disclosure relates to any one of the aforementioned methods, wherein the stabilized composite material is substantially dried for about 5 min., about 10 min., about 20 min., about 30 min., about 40 min., about 50 min., about 60 min., about 70 min., about 80 min., about 90 min., about 100 min., about 110 min., about 120 min., about 130 min., or about 140 min.

The disclosure relates to any one of the aforementioned methods, wherein the stabilized composite material is substantially dried at a temperature of about 20 °C, about 25 °C, about 30 °C, about 35 °C, about 40 °C, about 45 °C, about 50 °C, about 55 °C, or about 60 °C.

The disclosure relates to any one of the aforementioned methods, wherein the macroporous gel displays a selective interaction for the therapeutic agent.

The disclosure relates to any one of the aforementioned methods, wherein the macroporous gel displays a specific interaction for the therapeutic agent.

Disclosed herein is a method of delivering a therapeutic agent to a subject in need thereof, comprising the step of:

contacting any one of the aforementioned composite materials with a second solution, thereby dissociating the therapeutic agent from the composite material and forming a third solution; and
delivering the third solution to the subject.

The disclosure relates to any one of the aforementioned methods, wherein the second solution comprises a salt. The disclosure relates to any one of the aforementioned methods, wherein the second solution comprises a sodium salt. The disclosure relates to any one of the aforementioned methods, wherein the second solution comprises sodium chloride. The disclosure relates to any one of the aforementioned methods, wherein the concentration of the salt in the second solution is about 20 mM, about 30 mM, about 40 mM, about 50 mM, about 60 mM, about 70 mM, about 80 mM, about 90 mM, about 100 mM, about 110 mM, about 120 mM, about 130 mM, about 140 mM, about 150 mM, about 160 mM, about 170 mM, about 180 mM, about 190 mM, about 200 mM, about 210 mM, about 220 mM, about 230 mM, about 240 mM, about 250 mM, about 500 mM, about 750 mM, about 1 M, about 1.25 M, about 1.5 M, about 2 M, about 2.25 M, or about 2.5 M. The disclosure relates to any one of the aforementioned methods, wherein the concentration of the salt in the second solution is about 1 M.

The disclosure relates to any one of the aforementioned methods, wherein the second solution comprises a buffer salt. The disclosure relates to any one of the aforementioned methods, wherein the second solution comprises a phosphate. The disclosure relates to any one of the aforementioned methods, wherein the second solution comprises sodium phosphate. The disclosure relates to any one of the aforementioned methods, wherein the concentration of the buffer salt in the second solution is about 20 mM, about 30 mM, about 40 mM, about 50 mM, about 60 mM, about 70 mM, about 80 mM, about 90 mM, about 100 mM, about 110 mM, about 120 mM, about 130 mM, about 140 mM, about 150 mM, about 160 mM, about 170 mM, about 180 mM, about 190 mM, about 200 mM, about 210 mM, about 220 mM, about 230 mM, about 240 mM, or about 250 mM. The disclosure relates to any one of the aforementioned methods, wherein the concentration of the buffer salt in the second solution is about 100 mM.

The disclosure relates to any one of the aforementioned methods, wherein the pH of the first solution is about 4, about 4.5, about 5, about 5.5, about 6, about 6.5, about 6.6, about 6.7, about 6.8, about 6.9, about 7, about 7.1, about 7.2, about 7.3, about 7.4, about 7.5, or about 8. The disclosure relates to any one of the aforementioned methods, wherein the pH of the first solution is about 7.2.

The disclosure relates to any one of the aforementioned methods, wherein the composite material is configured in a syringe.

The disclosure relates to any one of the aforementioned methods, wherein the composite material is configured in an intravenous line.

The disclosure relates to any one of the aforementioned methods, wherein the composite material is configured in a vial.

The disclosure relates to any one of the aforementioned methods, wherein the third solution is delivered to the subject intravenously.

**EXEMPLIFICATION**

The following examples are provided to illustrate the invention. It will be understood, however, that the specific details given in each example have been selected for purpose of illustration and are not to be construed as limiting the scope of the invention. Generally, the experiments were conducted under similar conditions unless noted.

*Example 1 -Protein A membrane drying conditions*

Experiments were conducted with membranes covalently bound to protein A as proof that membranes with associated biomolecules may be adequately dried and stabilized against degradation, while maintaining their function upon rewetting. Biomolecules need not be covalently attached to the membrane for the principle to hold true.

To improve protein A stability after being immobilized onto the aldehyde membrane, the protein must be dried in the presence of "drying agent" that is capable of preserving the selectivity and activity of the protein A grafted molecule. Three options were considered and examined for drying protein A membrane as shown below.

**1.1 PEG/Glucose**

Polyethylene glycols, which are known as protein friendly macromolecules, have been used widely to preserve immobilized molecules on surfaces and inside polymers. Sugars, on the other hand, are well known for their positive impact as drying agent for lyophilized and immobilized proteins. Early work on drying ProA membrane showed that a mixture of PEG (10 wt%, avg. Mwt 2000 Da) and glucose (10 wt%) in 50 mM phosphate buffer maintained ~80% of protein A activity after drying for 2 hours in oven.

One of the problems associated with PEG is to achieve complete wash before the capturing process of IgG is conducted. Traces of PEG may interfere with the binding process and minimize the binding efficiency.

**1.2 Glycerol/Trehalose**

Beside its capability to preserve the three-dimensional structure of proteins, glycerol is easier to wash off the membrane compared to PEG. Trehalose, on the other hand, is a disaccharide and has been used to stabilize protein structure. Exploratory work showed that a ProA membrane dried with a mixture of glycerol (50 wt%) and trehalose (10 wt%) was capable of preserving immobilized protein A; the binding capacity of the "dried" membrane was ~95% of the wet membrane.

Despite its good performance, using glycerol can be questionable, as membranes may still have some water content (which may affect the protein stability in the long run), and installing a membrane in the device maybe associated with undesirable technical issues.

**1.3 Trehalose**

Trehalose, a disaccharide, is a protein-friendly molecule that can serve as drying agent for membranes. Drying a ProA membrane using trehalose proved to be successful, as the dried membrane exhibited binding capacity that ranged from 90-85% of the wet membrane binding capacity. Because of it is hydrophilic nature and small molecule size, it can be washed easily off the membrane, so it won't affect the IgG binding step.

ProA membrane was soaked for 2 h in 10 wt% trehalose in 0.1 M phosphate buffer at pH 7. Then, the membrane was dried in the oven (50 °C) for another 2 hours. This procedure was studied in some detail to determine the variability of the process and the factors that affect the efficiency of preserving protein A activity.

**1.3.1 Soaking time**

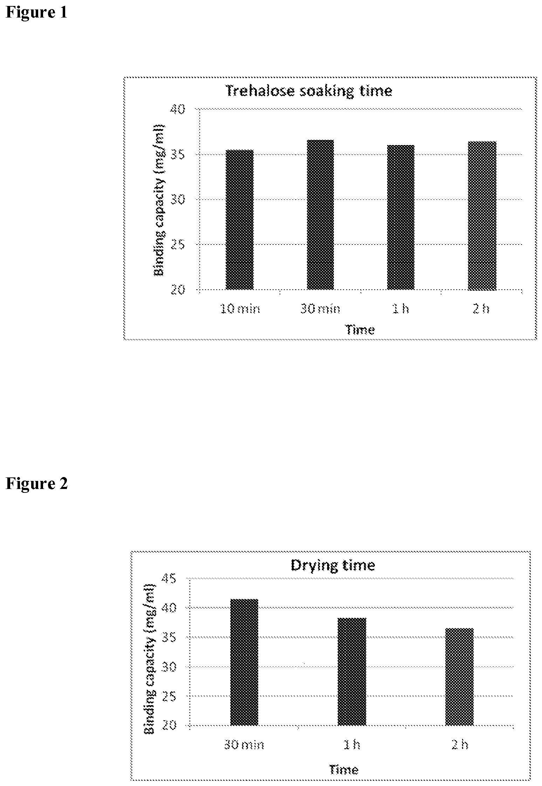

Shorter soaking time is advantageous in any manufacturing process. Examining soaking time of ProA membrane in trehalose solution prior to drying showed that it was possible to adopt soaking time as short as 10 minutes while preserving the binding activity of the membrane. [Membrane 090824-E2-AR9]. See Figure 1.

### 1.3.2 Drying time

Drying time can be critical in terms of manufacturing stable membranes that perform consistently. Long drying times put stress on the protein A moieties and may result in structural damages. Although short drying times are desired, incomplete drying (i.e. incomplete removal of water) can damage the membrane in the long run as the residual water molecules allow slow changes in protein structure and possibly result in denaturation. Therefore, any drying process should ensure adequate and efficient removal of water without overstressing the immobilized protein A.

A ProA membrane was dried for different time periods. Results showed the membrane can be dried effectively in 30 minutes in the oven (50 °C) and preserve its binding capability. See Figure 2. It is worth noting that drying conditions in manufacturing lines are different than the laboratory oven approach. More work is needed to explore drying process conditions in pilot/manufacturing environment. [Membrane 090824-E2-AR9]

### 1.3.3 Concentration effect

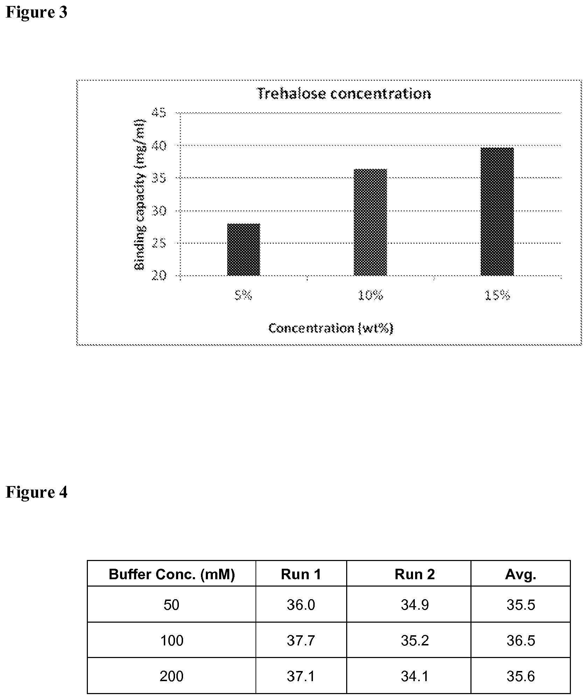

Trehalose concentration is a critical factor in the drying process, as it controls how much disaccharide can be picked up by the membrane and accordingly dictate the stability of the immobilized protein A. Three concentrations of trehalose were examined under regular conditions and, as expected, the process that employed higher concentration (15 wt%) resulted in the best results. [Membrane 090824-E2-AR9]. See Figure 3.

On the other hand, a low concentration of trehalose clearly showed that insufficient saccharide uptake was detrimental for the immobilized protein A (Figure 3). Therefore, any change in the process (like reducing soaking time) should ensure sufficient delivery of sugar into the membrane before drying; increasing the sugar concentration is considered the most viable option. More details can be found in section 1.3.5.

### 1.3.4 Buffer strength

Changing the ionic strength of the buffer that was used as a solvent for the trehalose did not affect the activity of the immobilized protein A. [Membrane 090824-E2-AR9]. See Figure 4.

### 1.3.5 Scooping experiments

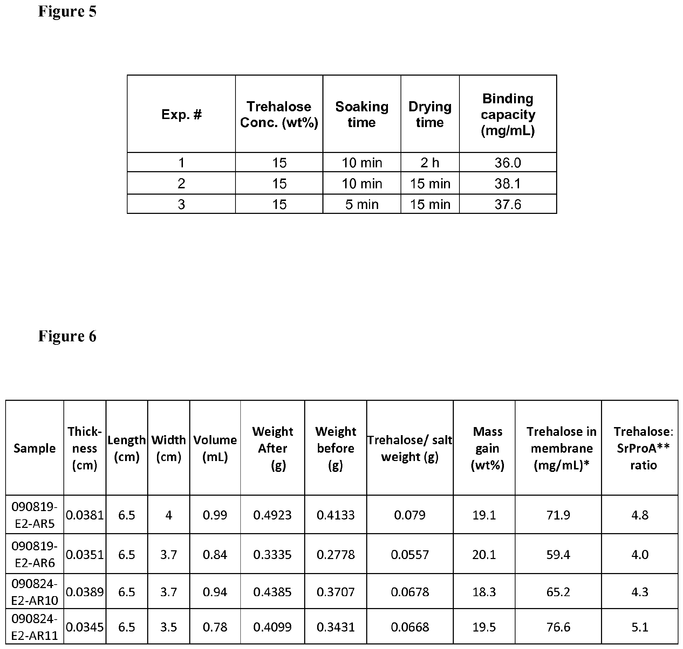

Limited experiments were carried out to scoop certain combinations of conditions in order to optimize the drying process and make it more convenient for the manufacturing line. Results, as shown in Figure 5, suggest that it is possible to adopt short drying time at the expense of trehalose concentration. [Membrane 090824-E2-AR9]

### 1.3.6 Mass gain and sugar/protein ratio

Common formulas for dried/lyophilized proteins usually contain sugar that is about 50 wt% of the dried protein. To ensure the stability of the protein, an adequate amount of sugar must be loaded into the membrane during the drying step. To examine the efficiency of current drying protocols, the quantity of trehalose in dried membranes was estimated by taking the difference between the mass of the membrane before and after drying. The amount of sugar was corrected assuming equal adsorption of buffer salt and sugar by the membrane, and the trehalose:protein ratio was calculated based on the assumption of 100% successful immobilization of protein A into the membrane (15 mg protein/mL membrane). Results show that, on average, the trehalose-to-protein ratio is about 4.5. Overall mass gain is ~ 19%. See Figure 6.

### *Example 2 - Protein A membrane and precursors membrane shelf life*

To determine the stability of ProA membrane and its precursors (aldehyde and epoxy), hand samples were made and stored at both room temperature and elevated temperature (accelerated aging), and stability was judged by both flux and binding capacity. Membranes stored at room temperature will be examined over a two-year time period (real time aging), while elevated temperature experiments will be capped at six weeks.

### 2.1 Protein A membrane

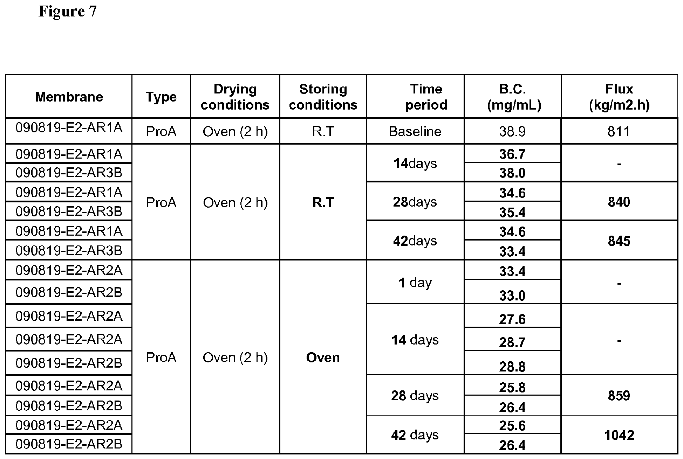

The available data showed that ProA membranes almost preserved their binding capacities over a period of 6 weeks when stored at room temperature. However, when the membrane was stored at 50 °C over the same time period, the binding capacity declined by 33%. It is worth noting that there was almost no change from 28 days to 42 days, which may suggest that the membrane may not undergo further changes in performance. See Figure 7.

For water flux, the membrane showed stable flux whether stored at room temperature or in the oven (50 °C). In addition, examining the buffer flux through ProA membrane showed that the membrane was insensitive to salt presence in the solution (data not shown).

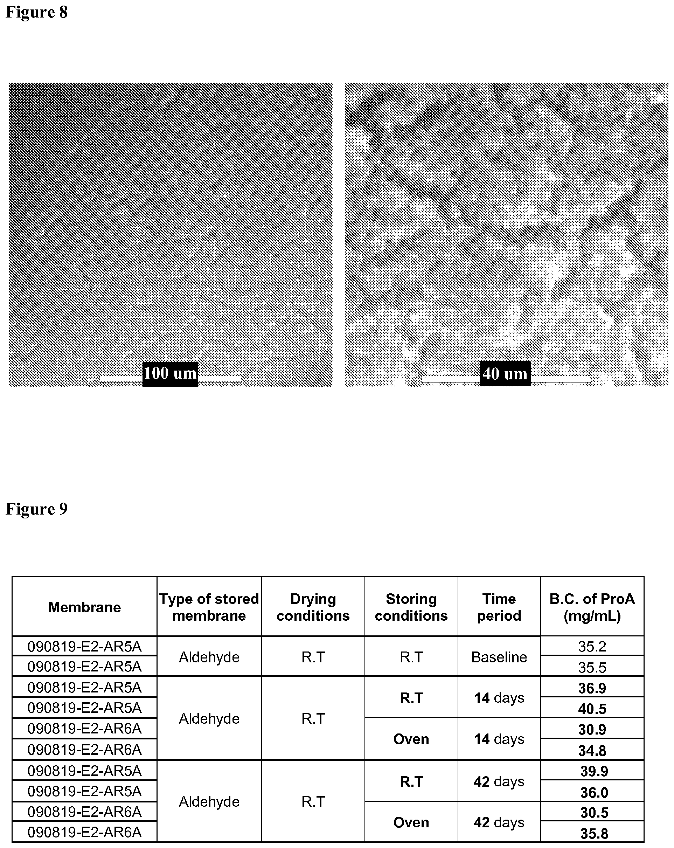

Examining the ProA membrane that was stored in the oven showed an interesting change in the structure, as many minor grooves and cracks developed just after 1 week of storage at 50 °C. See Figure 8.

## 2.2 Aldehyde membrane

To examine whether drying conditions can affect the aging of the aldehyde membrane, two series of aldehyde membranes were studied. The first set includes aldehyde membranes that were dried at room temperature then coupled with protein A. The second set includes aldehyde membranes that were dried in the oven (1 hour) at 50 °C and subsequently used for coupling. The stability/validity of the membranes was judged by both binding capacity and flux.

### 2.2.1 Aldehyde membrane dried at room temperature

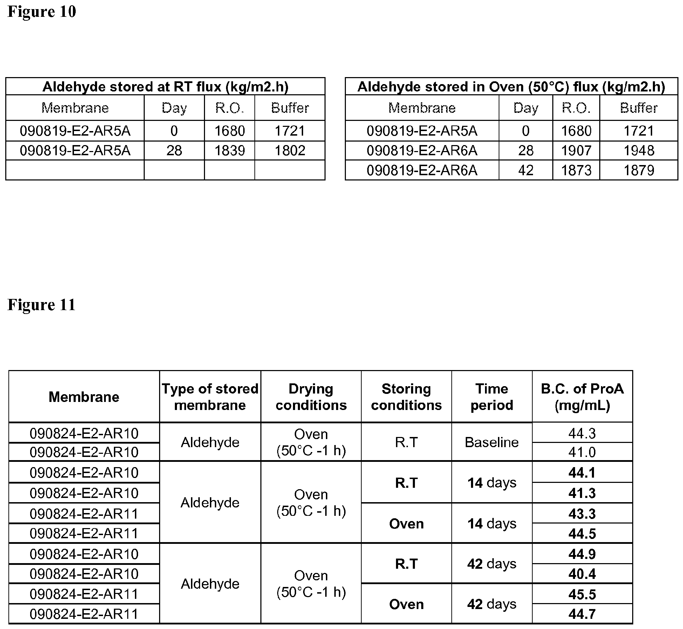

Examining the binding capacity of the stored membranes indicated a slightly higher binding capability of the membrane stored at room temperature compared to that stored in the oven at 50 °C. See Figure 9.
Examining the flux also showed that no drastic changes took place; differences in fluxes may be attributed to hand sample variability. See Figure 10.

### 2.2.2 Aldehyde membrane dried in oven (50 °C, 1 h)

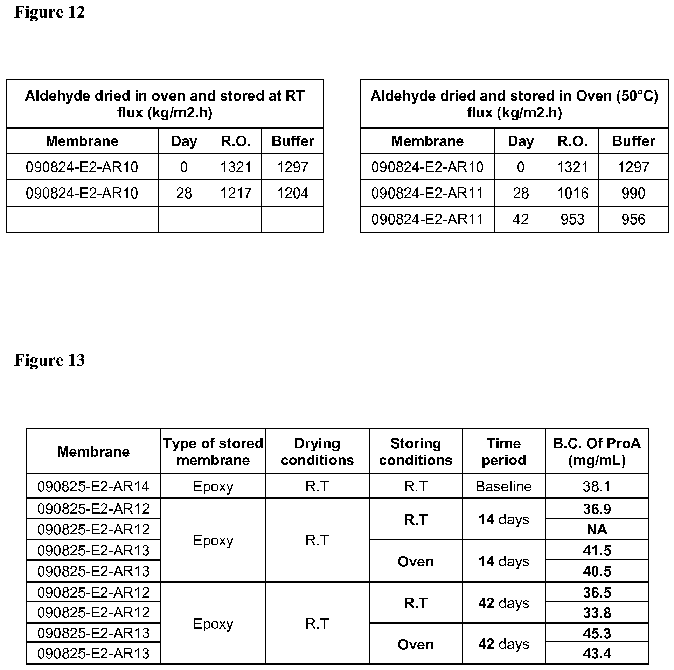

In general, the binding capacity results of the aging aldehyde membranes that were dried in the oven, regardless of the storing conditions, were found to be similar to (if not better than) the baseline sample (0 day). In fact, aldehyde membranes that were stored in the oven showed a slight increase in binding capacity, compared to those stored at room temperature. See Figure 11.
Interestingly, the flux of the aldehyde membrane stored in the oven was found to decline as the membrane aged, while the one stored at room temperature barely changed. It is possible that the slight increase of the binding capacity of the oven-stored membrane is associated with the flux decrease over time. See Figure 12.

## 2.3 Epoxy membrane

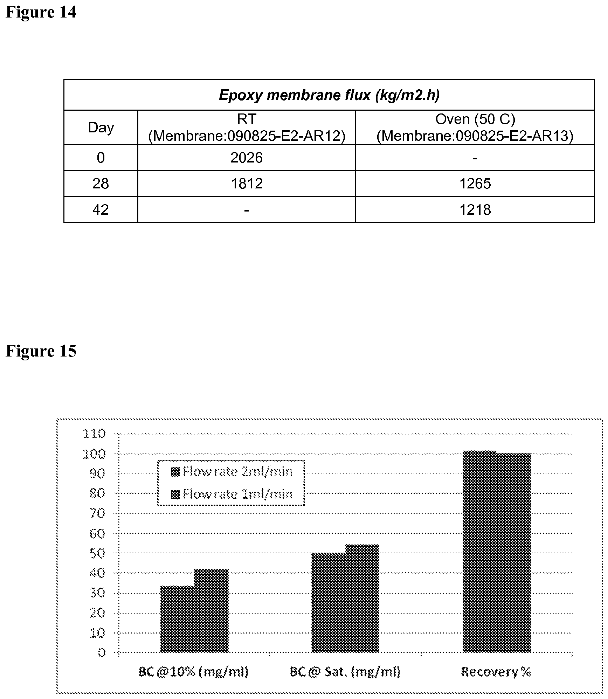

Results showed that the binding capacity of ProA derived from epoxy membranes that were stored at elevated temperatures were increasing as the membrane aged, while those stored at room temperature didn't change significantly. See Figure 13.
Examining the flux, on the other hand, showed that the flux decreased as the membrane aged in the oven (accelerated aging). The increase in the binding capacity could be associated with the flux decrease, and it may signal a membrane structural change that takes place over time. See Figure 14.

## Example 3 - Protein A membrane application conditions

ProA membranes are designed to selectively capture IgG. It is imperative to ensure selective binding and high recovery of IgG; therefore, work on optimizing the binding and elution conditions was carried out to determine the best conditions and the boundaries of parameters such as pH, flow rate, etc.

## 3.1 Binding conditions

Regular binding conditions were as follows: 20 mM phosphate buffer with 0.15 M NaCl at pH 7.4, flow rate = 1.0 mL/min.

### 3.1.1 Flow rate effect

Flow rate controls the amount of time that IgG molecules spend in contact with the membrane surface and, therefore, the binding efficiency. Low flow rates are not desirable for competition reasons and high flow rates can decrease the binding capacity.
To examine the flow rate effect, regular IgG binding experiments were carried out at two different flow rates and results are shown in Figure 15 (left bar = 2 mL/min; right bar = 1 mL/min) [Membrane 090824-E2-AR9].
Increasing the flow rate from 1.0 mL/min to 2.0 mL/min resulted in a decrease in binding capacity at low breakthrough (10%). However, the difference in binding capacities was smaller at high breakthrough (near the saturation). Recovery was not affected by the flow rate.

### 3.1.2 pH effect

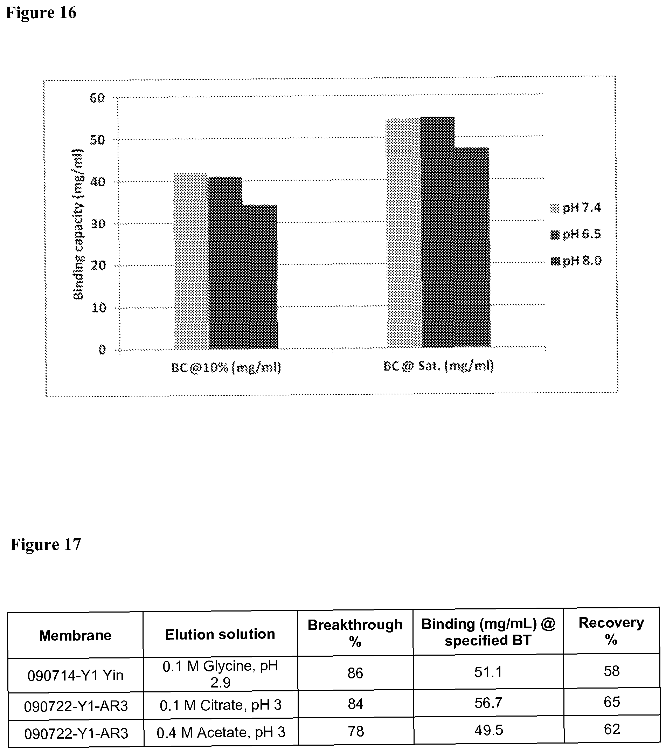

The binding forces between IgG and protein A are partly controlled by the pH of the solution. Therefore, it is useful to determine the "pH window" within which the membrane functions well. The pH of the binding solutions was varied from 6.5 up to 8.0 and the results are shown in Figure 16 [Membrane 090824-E2-AR9] (left bar = pH 7.4; middle bar = pH 6.5; right bar = pH 8.0).
It is obvious that binding was effective over the pH range 6.5 to 8.0. On the other hand, binding was lower at pH 8.0, whether at 10% breakthrough or at saturation.

## 3.2 Elution conditions

### 3.2.1 Regular elution with 0.1 M Glycine

Early work with ProA membranes showed that the recovery of IgG using the typical 0.1 M glycine solution at pH 3.0 was insufficient (65-80%). Increasing flow rate while eluting the IgG did not improve the elution.

### 3.2.2 Elution with acetate and citrate solution

Different buffer salts were tried in order to improve the recovery. Common buffers used with resins, such as citrate, were examined but didn't improve the recovery. Acetate was also examined (despite the fact it was shifted away from its pKa) and also exhibited poor performance (see Figure 17).

### 3.2.3 Elution with Glycine/Glucose/ethanol solution mixture

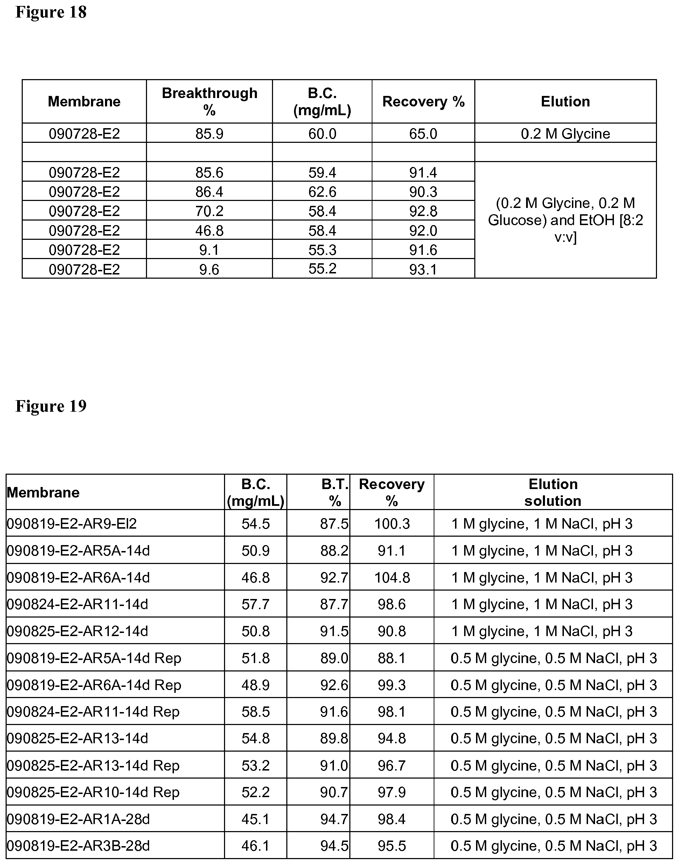

After many trials to improve the elution by using different buffer/additive systems, a mixture of glycine/glucose (0.2 M each) mixed with ethanol (8:2, V:V) showed very promising results. The recovery was higher than 90%, compared to original 0.1 M glycine solution or 0.2 M glycine. See Figure 18.

### 3.2.4 Elution with Glycine/NaCl solution

Despite the success of this mixture in eluting IgG from ProA membrane, using ethanol may constitute a challenge for users and may be incompatible with some industrial process. Therefore, there is a need to provide an alternative that eliminates the use of ethanol.

New eluting solutions that use high glycine and salt concentrations were examined and results showed very high recovery (Figure 19). The concentration of glycine can be reduced down to 0.5 M in the presence of 0.5 M NaCl without effecting elution efficiency.

### 3.2.5 pH effect on recovery

Acidic conditions can be detrimental for IgG. Since the elution step is typically carried out at low pH (2.5-3), it is useful to explore higher pHs that allow efficient recovery from a ProA membrane and can be gentler on the eluted IgG.

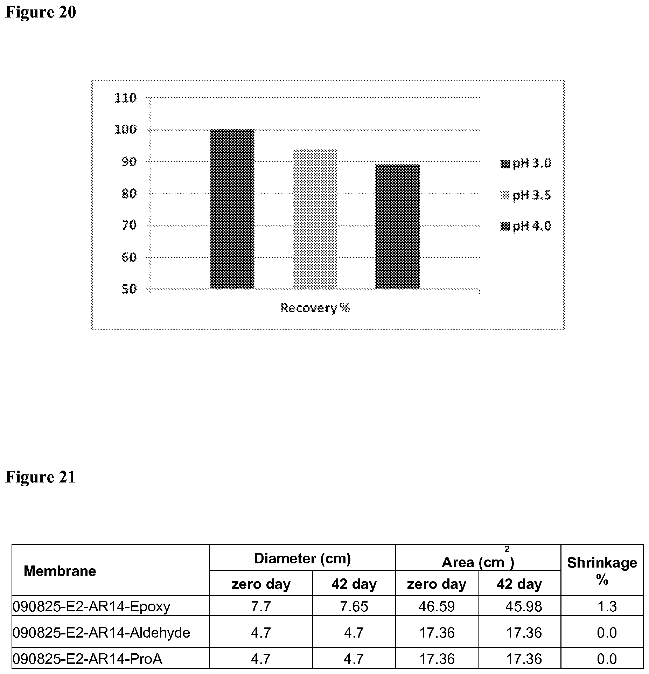

Binding/elution experiments were carried out using elution solutions of pH 3.0, 3.5, and 4.0. Results are shown in Figure 20 (left bar = pH 3.0; middle bar = pH 3.5; right bar = pH 4.0) and suggest that the pH of the elution solution should not exceed pH 3.5. [Membrane 090824-E2-AR9]

### *Example 4 - Protein A membrane mechanical properties*

### 4.1 Shrinkage during storing/manufacturing

### 4.1.1 Changes in Epoxy, Aldehyde, and ProA membrane on storing

To examine any possible changes that may happen to a ProA membrane (or its precursors) during storage, a well-defined area of the dried membrane (coupons), each 7.7 cm in diameter were cut from each type of the membrane and were stored in the oven (50 °C). Results (Figure 21) indicated that minimal changes took place after 6 weeks of storage.

### 4.1.2 Change in membrane dimensions as going from aldehyde to ProA membrane

It is important to confirm that the dimensions of the membranes are not going to change significantly during the transition from a membrane with aldehyde functionality to a membrane with ProA functionality.

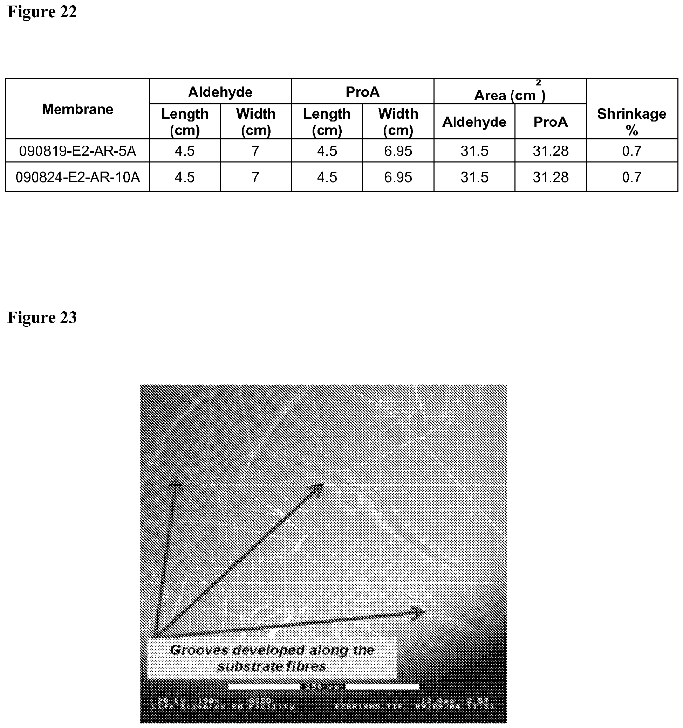

Two pieces of well-defined area were made in aldehyde form, then coupled with protein A. The surface area was measured before and after the coupling process, and changes were calculated. Results indicated minimal changes in area as shown in Figure 22.

### 4.2 Mechanical strength

To probe the strength of the membrane and its ability to withstand handling conditions, a piece of ProA membrane was rolled around a cylindrical body (1.35 cm in diameter) on one face. The membrane was then unwrapped and rolled around the cylinder on its opposite face.

Binding capacity of the wrapped membrane was examined (39.3 mg/mL) and was similar to the non-stressed baseline sample (38.1 mg/mL). However, ESEM examination showed that some grooves developed along the substrate fibres (Figure 23), suggesting that excessive handling of the membrane may generate structural defects. Therefore, membrane must be carefully handled to avoid generating defects. [Membrane 090825-E2-AR14]

### *Example 5 - IgG Physical immobilization on membrane*

**Objective:** Immobilize IgG on membrane, then elute it without loss in biological activity.

### Overview

    1) Immobilization

- Bind IgG protein on membrane using ion exchange conditions
- Wash unbound protein
- Determine amount of the captured protein on membrane
- Flow through buffer with stabilizer (pass/soak/pass)
- Dry at room temperature using air flow dryer

    2) Elution

- Wash membrane with binding buffer and determining protein leaching into washing solution
- Elute protein in elution buffer and determining protein content in the solution, then determining recovery %

3) Activity assessment

- Use IgG titer kit [Thermo (Pierce) PI-23310] that is based on protein activity to determine active protein content
- Determine the ratio of protein estimation using the kit to that determined using UV absorbance. This ratio represents the % of the active protein in the eluted protein.

## Experimental

**Immobilization step:** IgG lyophilized powder (Equitech Inc.) was used to made a solution of 0.5 mg/mL IgG in binding buffer (85 mM sodium acetate, pH 5). 30 mL of that solution was passed at 2 mL/min flow rate through a 25-mm disc of membrane, which was mounted on 25-mm holder. Subsequently, in order to wash unbound protein, 14 mL of binding buffer was passed through the membrane at 1 mL/min flow rate. The effluent solutions from the two steps (30 mL + 14 mL, binding and washing) were collected, and the absorbance at 280 nm was measured to determine the amount of protein present in the solutions. From the absorbance, the amount of protein captured on the membrane was calculated.

A stabilizer solution was made by dissolving 10 wt% of stabilizer in binding buffer (85 mM sodium acetate, pH 5), and 6 mL of that solution was passed through the membrane at 0.5 mL/min flow rate. The flow was stopped and the membrane was allowed to soak for 5 min in the stabilizer solution, after which another 6 mL of the stabilizer solution was passed through the membrane at 1 mL/min flow rate. The total passed solution (6 mL + 6 mL) was collected and UV absorbance (280 nm) was measured to determine any protein leached during this step.

The holder cell was taken apart and membrane disc was removed and mounted on stand, then dried at room temperature with the aid of air flow drier (10-15 min).

**Elution and activity assessment:** The dry membrane disc was placed onto the holder cell and wetted with few drops of binding solution (85 mM sodium acetate, pH 5) to ensure the proper placement of membrane was achieved. The holder was re-assembled and 12 mL of binding solution was passed through the cell to ensure complete removal of air bubbles. This solution was collected to determine whether any protein was leached during the washing step. Then, a quantity of 12-14 mL of elution solution (0.1 M sodium phosphate, 1 M NaCl, pH 7.2) was passed through the cell and this solution was collected. UV absorbance (280 nm) was measured in order to determine the concentration of protein, the total amount of the eluted protein, and recovery %.

Using an IgG titer kit (Thermo - Pierce PI-23310), which relies on antibody-antigen interaction for detection, the IgG protein concentration in the elution solution was determined. The standard was lyophilized IgG powder in solution.

The ratio of IgG concentration that was determined by activity assay, to the concentration based on UV absorbance represents the active ratio of the IgG protein in the elution solution.

As a control sample, an IgG powder was dissolved in buffer solution (0.1 M sodium phosphate, 0.15 M NaCl, pH 7.2), then dried over a glass Petri dish at room temperature with the aid of flowing air, and then dissolved again in buffer. Activity was then assessed.

## Results

While the hydrophilic non-charged nature of the stabilizer makes it unlikely that it will interfere with the ionic interactions between the membrane and the protein molecules, it is important to confirm that it will not lead to any protein leaching during the stabilization.

The stabilizer solutions that had been passed through the membrane showed no protein leaching. This shows that it is possible to apply the stabilizer solution without displacing the adsorbed protein from the membrane.

In order to elute proteins in conditions that favour the stability of the protein, the pH of the elution solution should be within the range of 6.5-7.5. At such pH, the elution is expected to be easier because both the protein molecules and the membrane will be more negatively charged.

Eluting IgG with solution of pH 7.2 was highly efficient; therefore it was possible to use this solution for eluting physically immobilized IgG from the membrane in conditions that are favorable for protein stability and maintaining protein activity.

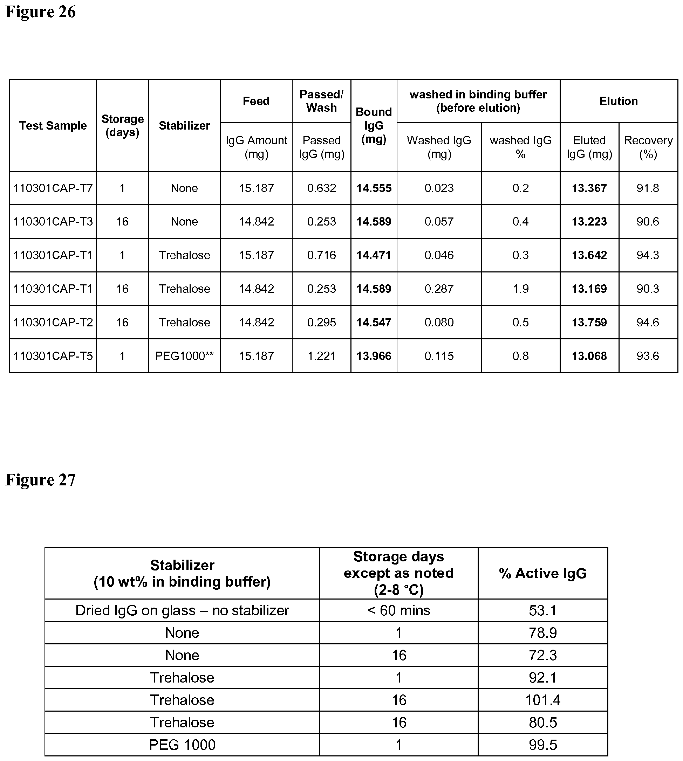

Binding and eluting results are shown in Figure 26.

To determine the biological stability of the eluted protein, after eluting IgG from the membranes, it was assayed as outlined in the kit procedure and the concentration of active protein molecules was determined. The ratio of this active protein to the total amount of protein (indicated by UV absorbance) can indicate the ratio of activity of the eluted protein. The IgG lyophilized powder was used as standard material from which a calibration curve was constructed.

To demonstrate the effect of both membrane and stabilizer on maintaining the protein bioactivity during the drying process, a control sample was made by drying an IgG solution over a glass Petri-dish at room temperature, with the aid of flowing air. The dried sample was reconstituted in buffer and the activity was determined similar to other samples.

Results suggest that IgG can be physically immobilized on a membrane and remain biologically active, especially when a stabilizer (such as trehalose or polyethylene glycol) is included in the drying process. See Figure 27.

### Conclusions

IgG protein can be captured and immobilized on a membrane and kept in dry conditions without major loss in protein activity. The presence of hydrophilic stabilizers during the drying process can preserve the protein stability. The stabilizing agent does not interfere with the physical embodiment of the protein. It is possible to use elution conditions at a pH different than the one used in the binding processes without affecting recovery of the protein.

### Appendix

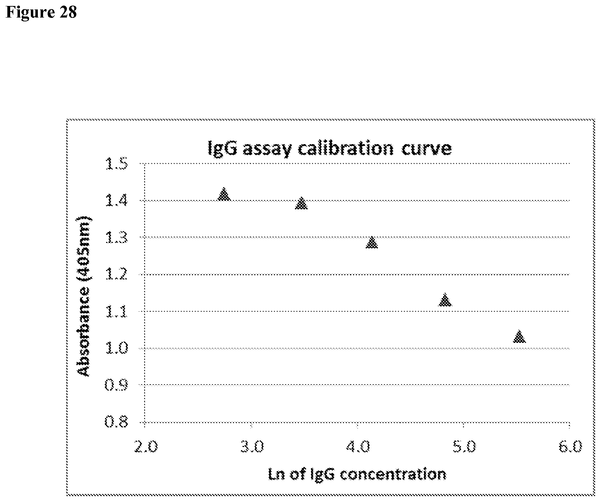

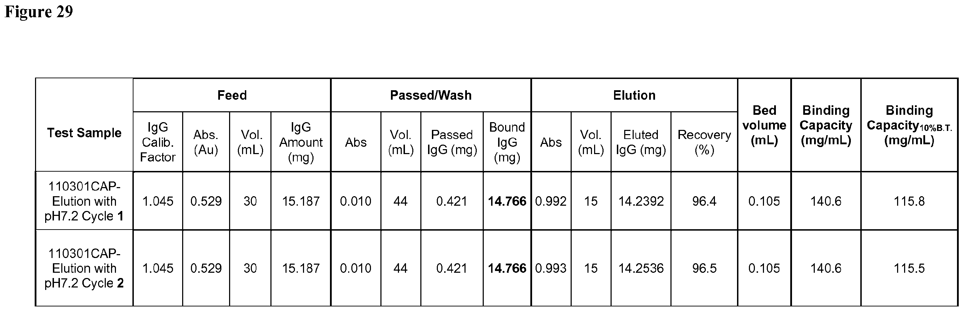

Figure 28 depicts IgG assay calibration curve. Additional and detailed results for elution using pH 7.2 buffer solution are shown in Figure 29.

### Claims

1. A dried, stabilized composite material, comprising:

   a support member, comprising a plurality of pores extending through the support member; a macroporous cross-linked gel, comprising a plurality of macropores; a protein covalently bonded to the macroporous cross-linked gel, wherein the protein is protein A; and a stabilizing agent comprising polyethylene glycol, glucose, glycerol or sucrose; wherein the macroporous cross-linked gel is located in the pores of the support member; and the average pore diameter of the macropores is less than the average pore diameter of the pores.

2. The dried, stabilized composite material of claim 1, wherein the stabilizing agent comprises polyethylene glycol.

3. The dried, stabilized composite material of claim 1, wherein the stabilizing agent comprises polyethylene glycol, glycerol or sucrose.

4. The dried, stabilized composite material of claim 1, wherein the stabilizing agent comprises glycerol.

5. The dried, stabilized composite material of any one of claims 1-4, further comprising a salt.

6. The dried, stabilized composite material of claim 5, wherein the salt is a phosphate salt or an acetate salt.

7. The dried, stabilized composite material of any one of claims 1-6, wherein the composite material is substantially stable at 20 °C, 25 °C, 30 °C, 35 °C, 40 °C, 45 °C, 50 °C, 55 °C, or 60 °C.

8. The dried, stabilized composite material of any one of claims 1-7, wherein the mass ratio of the stabilizing agent to Protein A is 10:1, 9:1, 8:1, 7:1, 6:1, 5:1, 4:1, 3:1, 2:1, 1:1, 1:2, or 1:4.

### Patentansprüche

1. Getrocknetes, stabilisiertes Verbundmaterial, umfassend:

   ein Trägerelement, umfassend eine Vielzahl von Poren, die sich durch das Trägerelement erstrecken; ein makroporöses vernetztes Gel, umfassend eine Vielzahl von Makroporen; ein Protein, das kovalent an das makroporöse vernetzte Gel gebunden ist, wobei das Protein Protein A ist; und ein Stabilisierungsmittel, umfassend Polyethylenglycol, Glucose, Glycerin oder Saccharose; wobei sich das makroporöse vernetzte Gel in den Poren des Trägerelements befindet; und der mittlere Porendurchmesser der Makroporen geringer als der mittlere Porendurchmesser der Poren ist.

2. Getrocknetes, stabilisiertes Verbundmaterial nach Anspruch 1, wobei das Stabilisierungsmittel Polyethylenglycol umfasst.

3. Getrocknetes, stabilisiertes Verbundmaterial nach Anspruch 1, wobei das Stabilisierungsmittel Polyethylenglycol, Glycerin oder Saccharose umfasst.

4. Getrocknetes, stabilisiertes Verbundmaterial nach Anspruch 1, wobei das Stabilisierungsmittel Glycerin umfasst.

5. Getrocknetes, stabilisiertes Verbundmaterial nach einem der Ansprüche 1-4, ferner umfassend ein Salz.

6. Getrocknetes, stabilisiertes Verbundmaterial nach Anspruch 5, wobei das Salz ein Phosphatsalz oder ein Acetatsalz ist.

7. Getrocknetes, stabilisiertes Verbundmaterial nach einem der Ansprüche 1-6, wobei das Verbundmaterial im Wesentlichen bei 20 °C, 25 °C, 30 °C, 35 °C, 40 °C, 45 °C, 50 °C, 55 °C oder 60°C stabil ist.

8. Getrocknetes, stabilisiertes Verbundmaterial nach einem der Ansprüche 1-7, wobei das Massenverhältnis vom Stabilisierungsmittel zum Protein A 10:1, 9:1, 8:1, 7:1, 6:1, 5:1, 4:1, 3:1, 2:1, 1:1, 1:2 oder 1:4 beträgt.

## Revendications

1. Matériau composite stabilisé, sec, comprenant :

   un membre de support, comprenant une pluralité de pores s'étendant à travers le membre de support ;
   un gel réticulé macroporeux, comprenant une pluralité de macropores ; une protéine liée de manière covalente au gel réticulé macroporeux, la protéine étant la Protéine A ;
   et un agent stabilisant comprenant du polyéthylène glycol, du glucose, du glycérol ou du saccharose ; le gel réticulé macroporeux étant situé dans les pores du membre de support ; et le diamètre de pore moyen des macropores est inférieur au diamètre moyen des pores.

2. Matériau composite stabilisé, sec de la revendication 1, dans lequel l'agent stabilisant comprend du polyéthylène glycol.

3. Matériau composite stabilisé, sec de la revendication 1, dans lequel l'agent stabilisant comprend du polyéthylène glycol, du glycérol ou du saccharose..

4. Matériau composite stabilisé, sec de la revendication 1, dans lequel l'agent stabilisant comprend du glycérol.

5. Matériau composite stabilisé, sec d'une quelconque des revendications 1 à 4, comprenant en outre un sel.

6. Matériau composite stabilisé, sec de la revendication 5, dans lequel le sel est un sel de phosphate ou un sel d'acétate.

7. Matériau composite stabilisé, sec d'une quelconque des revendications 1 à 6, le matériau composite étant sensiblement stable à 20 °C, 25 °C, 30 °C, 35 °C, 40 °C, 45 °C, 50 °C, 55 °C, ou 60 °C.

8. Matériau composite stabilisé, sec d'une quelconque des revendications 1 à 7, dans lequel le rapport massique de l'agent stabilisant à la Protéine A est de 10:1, 9:1, 8:1, 7:1, 6:1, 5:1, 4:1, 3:1, 2:1, 1:1, 1:2 ou 1:4.

**Figure 1**

**Figure 2**

**Figure 3**

Trehalose concentration

**Figure 4**

| Buffer Conc. (mM) | Run 1 | Run 2 | Avg. |
|---|---|---|---|
| 50 | 36.0 | 34.9 | 35.5 |
| 100 | 37.7 | 35.2 | 36.5 |
| 200 | 37.1 | 34.1 | 35.6 |

**Figure 5**

| Exp. # | Trehalose Conc. (wt%) | Soaking time | Drying time | Binding capacity (mg/mL) |
|---|---|---|---|---|
| 1 | 15 | 10 min | 2 h | 36.0 |
| 2 | 15 | 10 min | 15 min | 38.1 |
| 3 | 15 | 5 min | 15 min | 37.6 |

**Figure 6**

| Sample | Thick-ness (cm) | Length (cm) | Width (cm) | Volume (mL) | Weight After (g) | Weight before (g) | Trehalose/ salt weight (g) | Mass gain (wt%) | Trehalose in membrane (mg/mL)* | Trehalose: SrProA** ratio |
|---|---|---|---|---|---|---|---|---|---|---|
| 090819-E2-AR5 | 0.0381 | 6.5 | 4 | 0.99 | 0.4923 | 0.4133 | 0.079 | 19.1 | 71.9 | 4.8 |
| 090819-E2-AR6 | 0.0351 | 6.5 | 3.7 | 0.84 | 0.3335 | 0.2778 | 0.0557 | 20.1 | 59.4 | 4.0 |
| 090824-E2-AR10 | 0.0389 | 6.5 | 3.7 | 0.94 | 0.4385 | 0.3707 | 0.0678 | 18.3 | 65.2 | 4.3 |
| 090824-E2-AR11 | 0.0345 | 6.5 | 3.5 | 0.78 | 0.4099 | 0.3431 | 0.0668 | 19.5 | 76.6 | 5.1 |

**Figure 7**

| Membrane | Type | Drying conditions | Storing conditions | Time period | B.C. (mg/mL) | Flux (kg/m2.h) |
|---|---|---|---|---|---|---|
| 090819-E2-AR1A | ProA | Oven (2 h) | R.T | Baseline | 38.9 | 811 |
| 090819-E2-AR1A | ProA | Oven (2 h) | R.T | 14days | 36.7 | - |
| 090819-E2-AR3B | | | | | 38.0 | |
| 090819-E2-AR1A | | | | 28days | 34.6 | 840 |
| 090819-E2-AR3B | | | | | 35.4 | |
| 090819-E2-AR1A | | | | 42days | 34.6 | 845 |
| 090819-E2-AR3B | | | | | 33.4 | |
| 090819-E2-AR2A | ProA | Oven (2 h) | Oven | 1 day | 33.4 | - |
| 090819-E2-AR2B | | | | | 33.0 | |
| 090819-E2-AR2A | | | | 14 days | 27.6 | - |
| 090819-E2-AR2A | | | | | 28.7 | |
| 090819-E2-AR2B | | | | | 28.8 | |
| 090819-E2-AR2A | | | | 28 days | 25.8 | 859 |
| 090819-E2-AR2B | | | | | 26.4 | |
| 090819-E2-AR2A | | | | 42 days | 25.6 | 1042 |
| 090819-E2-AR2B | | | | | 26.4 | |

Figure 8

Figure 9

| Membrane | Type of stored membrane | Drying conditions | Storing conditions | Time period | B.C. of ProA (mg/mL) |
|---|---|---|---|---|---|
| 090819-E2-AR5A | Aldehyde | R.T | R.T | Baseline | 35.2 |
| 090819-E2-AR5A | | | | | 35.5 |
| 090819-E2-AR5A | Aldehyde | R.T | R.T | 14 days | 36.9 |
| 090819-E2-AR5A | | | | | 40.5 |
| 090819-E2-AR6A | | | Oven | 14 days | 30.9 |
| 090819-E2-AR6A | | | | | 34.8 |
| 090819-E2-AR5A | Aldehyde | R.T | R.T | 42 days | 39.9 |
| 090819-E2-AR5A | | | | | 36.0 |
| 090819-E2-AR6A | | | Oven | 42 days | 30.5 |
| 090819-E2-AR6A | | | | | 35.8 |

**Figure 10**

| Aldehyde stored at RT flux (kg/m2.h) | | | |
|---|---|---|---|
| Membrane | Day | R.O. | Buffer |
| 090819-E2-AR5A | 0 | 1680 | 1721 |
| 090819-E2-AR5A | 28 | 1839 | 1802 |
| | | | |

| Aldehyde stored in Oven (50°C) flux (kg/m2.h) | | | |
|---|---|---|---|
| Membrane | Day | R.O. | Buffer |
| 090819-E2-AR5A | 0 | 1680 | 1721 |
| 090819-E2-AR6A | 28 | 1907 | 1948 |
| 090819-E2-AR6A | 42 | 1873 | 1879 |

**Figure 11**

| Membrane | Type of stored membrane | Drying conditions | Storing conditions | Time period | B.C. of ProA (mg/mL) |
|---|---|---|---|---|---|
| 090824-E2-AR10 | Aldehyde | Oven (50°C -1 h) | R.T | Baseline | 44.3 |
| 090824-E2-AR10 | | | | | 41.0 |
| 090824-E2-AR10 | Aldehyde | Oven (50°C -1 h) | R.T | 14 days | 44.1 |
| 090824-E2-AR10 | | | | | 41.3 |
| 090824-E2-AR11 | | | Oven | 14 days | 43.3 |
| 090824-E2-AR11 | | | | | 44.5 |
| 090824-E2-AR10 | Aldehyde | Oven (50°C -1 h) | R.T | 42 days | 44.9 |
| 090824-E2-AR10 | | | | | 40.4 |
| 090824-E2-AR11 | | | Oven | 42 days | 45.5 |
| 090824-E2-AR11 | | | | | 44.7 |

**Figure 12**

| Aldehyde dried in oven and stored at RT flux (kg/m2.h) | | | |
|---|---|---|---|
| Membrane | Day | R.O. | Buffer |
| 090824-E2-AR10 | 0 | 1321 | 1297 |
| 090824-E2-AR10 | 28 | 1217 | 1204 |
| | | | |

| Aldehyde dried and stored in Oven (50°C) flux (kg/m2.h) | | | |
|---|---|---|---|
| Membrane | Day | R.O. | Buffer |
| 090824-E2-AR10 | 0 | 1321 | 1297 |
| 090824-E2-AR11 | 28 | 1016 | 990 |
| 090824-E2-AR11 | 42 | 953 | 956 |

**Figure 13**

| Membrane | Type of stored membrane | Drying conditions | Storing conditions | Time period | B.C. Of ProA (mg/mL) |
|---|---|---|---|---|---|
| 090825-E2-AR14 | Epoxy | R.T | R.T | Baseline | 38.1 |
| 090825-E2-AR12 | Epoxy | R.T | R.T | 14 days | 36.9 |
| 090825-E2-AR12 | | | | | NA |
| 090825-E2-AR13 | | | Oven | 14 days | 41.5 |
| 090825-E2-AR13 | | | | | 40.5 |
| 090825-E2-AR12 | Epoxy | R.T | R.T | 42 days | 36.5 |
| 090825-E2-AR12 | | | | | 33.8 |
| 090825-E2-AR13 | | | Oven | 42 days | 45.3 |
| 090825-E2-AR13 | | | | | 43.4 |

**Figure 14**

| Epoxy membrane flux (kg/m2.h) | | |
|---|---|---|
| Day | RT (Membrane:090825-E2-AR12) | Oven (50 C) (Membrane:090825-E2-AR13) |
| 0 | 2026 | - |
| 28 | 1812 | 1265 |
| 42 | - | 1218 |

**Figure 15**

Figure 16

Figure 17

| Membrane | Elution solution | Breakthrough % | Binding (mg/mL) @ specified BT | Recovery % |
|---|---|---|---|---|
| 090714-Y1 Yin | 0.1 M Glycine, pH 2.9 | 86 | 51.1 | 58 |
| 090722-Y1-AR3 | 0.1 M Citrate, pH 3 | 84 | 56.7 | 65 |
| 090722-Y1-AR3 | 0.4 M Acetate, pH 3 | 78 | 49.5 | 62 |

Figure 18

| Membrane | Breakthrough % | B.C. (mg/mL) | Recovery % | Elution |
|---|---|---|---|---|
| 090728-E2 | 85.9 | 60.0 | 65.0 | 0.2 M Glycine |
| | | | | |
| 090728-E2 | 85.6 | 59.4 | 91.4 | (0.2 M Glycine, 0.2 M Glucose) and EtOH [8:2 v:v] |
| 090728-E2 | 86.4 | 62.6 | 90.3 | |
| 090728-E2 | 70.2 | 58.4 | 92.8 | |
| 090728-E2 | 46.8 | 58.4 | 92.0 | |
| 090728-E2 | 9.1 | 55.3 | 91.6 | |
| 090728-E2 | 9.6 | 55.2 | 93.1 | |

Figure 19

| Membrane | B.C. (mg/mL) | B.T. % | Recovery % | Elution solution |
|---|---|---|---|---|
| 090819-E2-AR9-El2 | 54.5 | 87.5 | 100.3 | 1 M glycine, 1 M NaCl, pH 3 |
| 090819-E2-AR5A-14d | 50.9 | 88.2 | 91.1 | 1 M glycine, 1 M NaCl, pH 3 |
| 090819-E2-AR6A-14d | 46.8 | 92.7 | 104.8 | 1 M glycine, 1 M NaCl, pH 3 |
| 090824-E2-AR11-14d | 57.7 | 87.7 | 98.6 | 1 M glycine, 1 M NaCl, pH 3 |
| 090825-E2-AR12-14d | 50.8 | 91.5 | 90.8 | 1 M glycine, 1 M NaCl, pH 3 |
| 090819-E2-AR5A-14d Rep | 51.8 | 89.0 | 88.1 | 0.5 M glycine, 0.5 M NaCl, pH 3 |
| 090819-E2-AR6A-14d Rep | 48.9 | 92.6 | 99.3 | 0.5 M glycine, 0.5 M NaCl, pH 3 |
| 090824-E2-AR11-14d Rep | 58.5 | 91.6 | 98.1 | 0.5 M glycine, 0.5 M NaCl, pH 3 |
| 090825-E2-AR13-14d | 54.8 | 89.8 | 94.8 | 0.5 M glycine, 0.5 M NaCl, pH 3 |
| 090825-E2-AR13-14d Rep | 53.2 | 91.0 | 96.7 | 0.5 M glycine, 0.5 M NaCl, pH 3 |
| 090825-E2-AR10-14d Rep | 52.2 | 90.7 | 97.9 | 0.5 M glycine, 0.5 M NaCl, pH 3 |
| 090819-E2-AR1A-28d | 45.1 | 94.7 | 98.4 | 0.5 M glycine, 0.5 M NaCl, pH 3 |
| 090819-E2-AR3B-28d | 46.1 | 94.5 | 95.5 | 0.5 M glycine, 0.5 M NaCl, pH 3 |

**Figure 20**

**Figure 21**

| Membrane | Diameter (cm) | | Area (cm$^2$) | | Shrinkage % |
|---|---|---|---|---|---|
| | zero day | 42 day | zero day | 42 day | |
| 090825-E2-AR14-Epoxy | 7.7 | 7.65 | 46.59 | 45.98 | 1.3 |
| 090825-E2-AR14-Aldehyde | 4.7 | 4.7 | 17.36 | 17.36 | 0.0 |
| 090825-E2-AR14-ProA | 4.7 | 4.7 | 17.36 | 17.36 | 0.0 |

Figure 22

| Membrane | Aldehyde | | ProA | | Area (cm$^2$) | | Shrinkage % |
|---|---|---|---|---|---|---|---|
| | Length (cm) | Width (cm) | Length (cm) | Width (cm) | Aldehyde | ProA | |
| 090819-E2-AR-5A | 4.5 | 7 | 4.5 | 6.95 | 31.5 | 31.28 | 0.7 |
| 090824-E2-AR-10A | 4.5 | 7 | 4.5 | 6.95 | 31.5 | 31.28 | 0.7 |

Figure 23

Figure 24

| Membrane | Type | Drying conditions | Storing conditions | Time period | B.C. (mg/ml) |
|---|---|---|---|---|---|
| E2-AR1A | ProA | Oven (2 h) | R.T | Baseline | 38.9 |
| E2-AR1A | | | | 14 d | 36.7 |
| E2-AR3B | | | | | 38.0 |
| E2-AR1A | | | | 28 d | 34.6 |
| E2-AR3B | | | | | 35.4 |
| E2-AR1A | | | | 42 d | 34.6 |
| E2-AR3B | | | | | 33.4 |
| E2-AR1A | ProA | Oven (2 h) | R.T | 90 d | 39.9 |
| E2-AR3B | | | | | 36.6 |
| E2-AR3A | | | | 180 d | 33.5 |
| E2-AR3B | | | | | 34.3 |
| E2-AR3A | | | | 360 d | 33.1 |
| E2-AR3B | | | | | 33.3 |
| E2-AR1A | | | | 720 d | 25.5 |
| E2-AR3B | | | | | 26.1 |
| E2-AR2A | | | | 1 d | 33.4 |
| E2-AR2B | | | | | 33.0 |
| E2-AR2A | | | | 14 d | 27.6 |
| E2-AR2A Rep | ProA | Oven (2 h) | Oven | | 28.7 |
| E2-AR2B | | | | | 28.8 |
| E2-AR2A | | | | 28 d | 25.8 |
| E2-AR2B | | | | | 26.4 |
| E2-AR2A | | | | 42 d | 25.6 |
| E2-AR2B | | | | | 26.4 |

**Figure 25**

**(a)**

*ProA membrane stored at R.T.*

**(b)**

*ProA membrane (50C)*

## Figure 26

| Test Sample | Storage (days) | Stabilizer | Feed | | Passed/ Wash | Bound IgG (mg) | washed in binding buffer (before elution) | | Elution | |
|---|---|---|---|---|---|---|---|---|---|---|
| | | | IgG Amount (mg) | Passed IgG (mg) | | | Washed IgG (mg) | washed IgG % | Eluted IgG (mg) | Recovery (%) |
| 110301CAP-T7 | 1 | None | 15.187 | 0.632 | | **14.555** | 0.023 | 0.2 | **13.367** | 91.8 |
| 110301CAP-T3 | 16 | None | 14.842 | 0.253 | | **14.589** | 0.057 | 0.4 | **13.223** | 90.6 |
| 110301CAP-T1 | 1 | Trehalose | 15.187 | 0.716 | | **14.471** | 0.046 | 0.3 | **13.642** | 94.3 |
| 110301CAP-T1 | 16 | Trehalose | 14.842 | 0.253 | | **14.589** | 0.287 | 1.9 | **13.169** | 90.3 |
| 110301CAP-T2 | 16 | Trehalose | 14.842 | 0.295 | | **14.547** | 0.080 | 0.5 | **13.759** | 94.6 |
| 110301CAP-T5 | 1 | PEG1000** | 15.187 | 1.221 | | **13.966** | 0.115 | 0.8 | **13.068** | 93.6 |

## Figure 27

| Stabilizer (10 wt% in binding buffer) | Storage days except as noted (2-8 °C) | % Active IgG |
|---|---|---|
| Dried IgG on glass – no stabilizer | < 60 mins | 53.1 |
| None | 1 | 78.9 |
| None | 16 | 72.3 |
| Trehalose | 1 | 92.1 |
| Trehalose | 16 | 101.4 |
| Trehalose | 16 | 80.5 |
| PEG 1000 | 1 | 99.5 |

**Figure 28**

# Figure 29

| Test Sample | Feed | | | | Passed/Wash | | | | Elution | | | | Bed volume (mL) | Binding Capacity (mg/mL) | Binding Capacity$_{10\%B.T.}$ (mg/mL) |
|---|---|---|---|---|---|---|---|---|---|---|---|---|---|---|---|
| | IgG Calib. Factor | Abs. (Au) | Vol. (mL) | IgG Amount (mg) | Abs | Vol. (mL) | Passed IgG (mg) | Bound IgG (mg) | Abs | Vol. (mL) | Eluted IgG (mg) | Recovery (%) | | | |
| 110301CAP-Elution with pH7.2 Cycle **1** | 1.045 | 0.529 | 30 | 15.187 | 0.010 | 44 | 0.421 | **14.766** | 0.992 | 15 | 14.2392 | 96.4 | 0.105 | 140.6 | 115.8 |
| 110301CAP-Elution with pH7.2 Cycle **2** | 1.045 | 0.529 | 30 | 15.187 | 0.010 | 44 | 0.421 | **14.766** | 0.993 | 15 | 14.2536 | 96.5 | 0.105 | 140.6 | 115.5 |

EP 2 804 634 B1

**REFERENCES CITED IN THE DESCRIPTION**

**Patent documents cited in the description**

- US 2010178210 A
- US 2009029438 A
- US 7316919 B
- US 20080314831 A
- US 20080312416 A
- US 20090029438 A
- US 20090032463 A
- US 20090008328 A
- US 20090035552 A
- US 20100047551 A
- US 20100044316 A
- US 20080017578 A
- US 20110253616 A